# EUROPEAN PATENT APPLICATION

(11) **EP 4 538 290 A1**
(43) Date of publication of application: **16.04.2025**
(21) Application number: 23820161.0
(22) Date of filing: 12.06.2023
(51) Int. Cl.: C07K 16/28, C07K 14/54, C12N 15/62, A61P 37/04, A61P 35/00, A61K 38/00, A61P 37/00, C07K 14/715, A61K 39/00

(54) **FUSION PROTEIN COMPRISING ANTIBODY BINDING SPECIFICALLY TO TIGIT AND INTERLEUKIN-15, AND USE THEREOF**

(30) Priority: 10.06.2022 KR 20220070914; 05.09.2022 KR 20220112314; 18.01.2023 KR 20230007617
(71) Applicant: MediMabbio Inc., Seongnam-si, Gyeonggi-do 13449 (KR)
(72) Inventor: KANG, Yuhoi, Seongnam-si, Gyeonggi-do 13449 (KR); JO, Hongseok, Seongnam-si, Gyeonggi-do 13449 (KR)
(74) Representative: Gevers Patents
(86) International application number: PCT/KR2023/008061
(87) International publication number: WO 2023/239226

(57) **Abstract**

Provided are a fusion protein comprising an anti-TIGIT antibody binding specifically to TIGIT or an antigen-binding fragment thereof and interleuckin-15 (IL-15), and a medicinal use of the fusion protein.

## Description

### Technical Field

### Cross-Reference to Related Applications

This application claims the benefit of and priority to Korean Patent Application No. 10-2022-0070914, filed on June 10, 2022, Korean Patent Application No. 10-2022-0112314, filed on September 5, 2022, and Korean Patent Application No. 10-2023-0007617, filed on January 18, 2023, the entire contents of which are incorporated herein by reference.

The present disclosure relates to a fusion protein including an anti-TIGIT antibody or an antigen-binding fragment thereof that binds specifically to TIGIT, and interleukin-15 (IL-15), and a pharmaceutical use of the fusion protein.

### Background Art

Cancer immunotherapy is a method of treating cancer by utilizing the body's immune responses, and it is referred to as the fourth cancer treatment method following chemotherapy, surgery, and radiation therapy. Cancer immunotherapy requires mechanisms that enhance the recognition and response to tumor cells by activating the immune system. Immune system activation involves complex mechanisms, including the function of various cells such as antigen-presenting cells, which are essential for initiating antigen-specific responses, and effector cells, which are responsible for the destruction of tumor cells.

A representative example of these effector cells is cytotoxic T cells.

TIGIT (T cell immunoglobulin and ITIM (immunoreceptor tyrosine-based inhibition motif) domain), also referred to as WUCAM, VSIG9, or Vstm3, is a co-inhibitory receptor predominantly expressed in NK cells, CD8+ and CD4+ T cells, as well as regulatory T cells (Treg). TIGIT is a transmembrane protein including an ITIM domain, a transmembrane domain, and an immunoglobulin variable domain.

TIGIT expression is increased in disease environments such as tumor-infiltrating lymphocytes (TILs) and infections. TIGIT expression can be a hallmark for exhausted T cells, which exhibit lower effector functions compared to TIGIT-negative cells. Moreover, Treg cells expressing TIGIT exhibit enhanced immunosuppressive activity compared to TIGIT-negative Treg populations.

Based on these properties, drugs acting as antagonists to TIGIT (e.g., antagonistic anti-TIGIT antibodies) are expected to induce immune system activation, enhance immune responses in disease states such as cancer, and consequently have a beneficial effect on disease treatment.

### Disclosure

### Technical Problem

One aspect provides a fusion protein including: (1) an anti-TIGIT antibody or an antigen-binding fragment thereof that specifically recognizes TIGIT; and (2) IL-15.

In the fusion protein according to an embodiment, IL-15 may be linked to a C-terminus and/or N-terminus of the heavy chain (heavy chain variable region or heavy chain constant region) or light chain (light chain variable region or light chain constant region) of the anti-TIGIT antibody or antigen-binding fragment thereof, either through a linker or directly without a linker.

Another aspect provides a nucleic acid molecule encoding the fusion protein.

Another aspect provides a recombinant vector carrying the nucleic acid molecule encoding the fusion protein.

Another aspect provides a recombinant cell anchoring the nucleic acid molecule or the recombinant vector thereat.

Another aspect provides an immunopotentiator or an immunopotentiating pharmaceutical composition including, as an active ingredient, at least one selected from the group consisting of the fusion protein, a nucleic acid molecule encoding the fusion protein, a recombinant vector carrying the nucleic acid molecule, and a recombinant cell anchoring the nucleic acid molecule or recombinant vector thereat.

Another aspect provides a pharmaceutical composition for the prevention and/or treatment of immune-related diseases, including, as an active ingredient, at least one selected from the group consisting of the fusion protein, a nucleic acid molecule encoding the fusion protein, a recombinant vector carrying the nucleic acid molecule, and a recombinant cell anchoring the nucleic acid molecule or recombinant vector thereat.

Another aspect provides an anticancer agent or a pharmaceutical composition for the prevention and/or treatment of cancer, the agent or composition including, as an active ingredient, at least one selected from the group consisting of the fusion protein, a nucleic acid molecule encoding the fusion protein, a recombinant vector carrying the nucleic acid molecule, and a recombinant cell anchoring the nucleic acid molecule or recombinant vector thereat.

Another aspect provides a method for potentiating immunity, the method including a step of administering a pharmaceutically effective amount of at least one selected from the group consisting of the fusion protein, a nucleic acid molecule encoding the fusion protein, a recombinant vector carrying the nucleic acid molecule, and a recombinant cell anchoring the nucleic acid molecule or recombinant vector thereat to a subject in need of immune potentiation.

Another aspect provides a method for the prevention and/or treatment of immune-related diseases, the method including a step of administering a pharmaceutically effective amount of one or more selected from the group consisting of the fusion protein, a nucleic acid molecule encoding the fusion protein, a recombinant vector carrying the nucleic acid molecule, and a recombinant cell comprising the nucleic acid molecule or recombinant vector to a subject in need of prevention and/or treatment of immune-related diseases.

Another aspect provides a method for the prevention and/or treatment of cancer, the method including a step of administering a pharmaceutically effective amount of at least one selected from the group consisting of the fusion protein, a nucleic acid molecule encoding the fusion protein, a recombinant vector carrying the nucleic acid molecule, and a recombinant cell anchoring the nucleic acid molecule or recombinant vector thereat to a subject in need of prevention and/or treatment of cancer.

Another aspect provides a use of at least one selected from the group consisting of the fusion protein, a nucleic acid molecule encoding the fusion protein, a recombinant vector carrying the nucleic acid molecule, and a recombinant cell anchoring the nucleic acid molecule or recombinant vector thereat, for enhancing immunity or for the preparation of immunopotentiators.

Another aspect provides a use of at least one selected from the group consisting of the fusion protein, a nucleic acid molecule encoding the fusion protein, a recombinant vector carrying the nucleic acid molecule, and a recombinant cell anchoring the nucleic acid molecule or recombinant vector thereat, for the prevention and/or treatment of immune-related diseases or for the manufacture of drugs for the prevention and/or treatment of immune-related diseases.

Another aspect provides the use of at least one selected from the group consisting of the fusion protein, a nucleic acid molecule encoding the fusion protein, a recombinant vector carrying the nucleic acid molecule, and a recombinant cell anchoring the nucleic acid molecule or recombinant vector thereat, for the prevention and/or treatment of cancer or for the manufacture of drugs for the prevention and/or treatment of cancer.

### Technical Solution

Provided herein are a fusion protein including: an anti-TIGIT antibody or an antigen-binding fragment thereof that binds to TIGIT; and IL-15, as well as a pharmaceutical use thereof. The fusion protein exhibits (1) an activity of blocking the function of TIGIT by the anti-TIGIT antibody or an antigen-binding fragment thereof, thereby activating the immune system (e.g., enhancing effector T cell function, regulating Treg activity, increasing cytokine secretion, etc.), and (2) the advantage of high binding affinity to receptors and low immunogenicity due to IL-15, finding applications as diverse immunoactivators and/or immunotherapeutic agents.

Below, a detailed description will be given of the present disclosure.

### Definition of Terms

As used herein, the phrase that a nucleic acid molecule (which may be used interchangeably with "polynucleotide" or "gene") or a polypeptide (which may be used interchangeably with "protein") "comprises a specific nucleic acid sequence or amino acid sequence", "consists of a specific nucleic acid sequence or amino acid sequence", or "is represented by a specific nucleic acid sequence or amino acid sequence" means that the nucleic acid molecule or polypeptide essentially includes the specific nucleic acid sequence or amino acid sequence, and can be interpreted to include "substantially equivalent sequences" with variations (e.g., deletions, substitutions, modifications, and/or additions) in the specific nucleic acid sequence or amino acid sequence (or not excluding such variations from the specific nucleic acid sequence or amino acid sequence), as long as the original function and/or intended function of the nucleic acid molecule or polypeptide is maintained.

In an embodiment, the nucleic acid sequences or amino acid sequences provided herein may include mutants obtained by conventional mutagenesis methods, such as directed evolution or site-directed mutagenesis, insofar as the mutants retain the original function or desired function of the sequence. In an embodiment, when a nucleic acid molecule or polypeptide "comprises or consists of a specific nucleic acid sequence or amino acid sequence", this means that the nucleic acid molecule or polypeptide (i) essentially includes the specific nucleic acid sequence or amino acid sequence, or (ii) consists essentially of or essentially includes an amino acid sequence having 60% or greater, 70% or greater, 80% or greater, 85% or greater, 90% or greater, 91% or greater, 92% or greater, 93% or greater, 94% or greater, 95% or greater, 96% or greater, 97% or greater, 98% or greater, 99% or greater, 99.5% or greater, or 99.9% or greater homology with the specific nucleic acid sequence or amino acid sequence, while retaining its original function and/or desired function.

The nucleic acid sequences described herein may undergo various modifications within the coding region to reflect the codon preferences of the microorganism used for protein expression, as long as the amino acid sequence and/or function of the expressed protein remains unchanged due to the degeneracy of codons.

As used herein, the term "homology" refers to the degree of similarity between a given nucleic acid sequence or amino acid sequence and another sequence, and can be expressed as a percentage (%). Homology for nucleic acid sequences can be determined using well-known algorithms such as BLAST or FASTA, and programs like BLASTN or BLASTX have been developed based on the BLAST algorithm (see: http://www.ncbi.nlm.nih.gov).

### Fusion Protein

According to an aspect thereof, the present disclosure provides a fusion protein including: (1) an anti-TIGIT antibody or an antigen-binding fragment thereof that specifically recognizes TIGIT; and (2) IL-15.

In the fusion protein, the anti-TIGIT antibody or antigen-binding fragment thereof may have an activity that blocks the function of TIGIT to activate the immune system (e.g., enhancing effector T cell function, regulating Treg activity, increasing cytokine secretion, etc.). In addition, IL-15 is a safe polypeptide with high binding affinity to receptors and low immunogenicity, providing excellent immune potentiation effects while minimizing the risk of immune responses recognizing it as an antigen when administered in vivo. Therefore, the fusion protein can be applied as diverse immunopotentiators and/or immunotherapeutic agents.

In the fusion protein according to an embodiment, IL-15 may be linked to the C-terminus and/or N-terminus of a heavy chain (heavy chain variable region or heavy chain constant region) or light chain (light chain variable region or light chain constant region) of the anti-TIGIT antibody or antigen-binding fragment thereof, either through a linker or directly without a linker.

More specifically, the fusion protein may include: (1) an anti-TIGIT antibody (e.g., an IgG type antibody including two heavy chains and two light chains) and (2) one IL-15, where the IL-15 is linked, either through a peptide linker or directly without a peptide linker, to the C-terminus (constant region C-terminus) or N-terminus of either of the two heavy chains or either of the two light chains of the anti-TIGIT antibody.

In another embodiment, the fusion protein may include: (1) an anti-TIGIT antibody (e.g., an IgG type antibody including two heavy chains and two light chains); and (2) two IL-15 molecules, where each IL-15 is linked, either through a linker or directly without a linker, to the C-terminus or N-terminus of either of the two heavy chains or either of the two light chains of the anti-TIGIT antibody.

For instance, in the fusion protein:
(i) one of the two IL-15 molecules may be linked, either through a linker or directly without a linker, to the C-terminus (i.e., the C-terminus of the constant region (e.g., Fc)) or N-terminus (i.e., the N-terminus of the variable region) of one of the two heavy chains of the anti-TIGIT antibody, and the other IL-15 molecule may be linked, either through a linker or directly without a linker, to the C-terminus (i.e., the C-terminus of the constant region (e.g., Fc)) or N-terminus (i.e., the N-terminus of the variable region) of the other heavy chain of the anti-TIGIT antibody, or
(ii) one of the two IL-15 molecules may be linked, either through a linker or directly without a linker, to the C-terminus (i.e., the C-terminus of the constant region) or N-terminus (i.e., the N-terminus of the variable region) of one of the two light chains of the anti-TIGIT antibody, and the other IL-15 molecule may be linked, either through a linker or directly without a linker, to the C-terminus (i.e., the C-terminus of the constant region) or N-terminus (i.e., the N-terminus of the variable region) of the other light chain of the anti-TIGIT antibody.

More specifically, in the fusion protein:
(i) one of the two IL-15 molecules may be linked, either through a linker or directly without a linker, to the C-terminus of one Fc region of the two heavy chains of the anti-TIGIT antibody, and the other may be linked, either through a linker or directly without a linker, to the C-terminus of the other Fc region of the anti-TIGIT antibody, or
(ii) one of the two IL-15 molecules may be linked, either through a linker or directly without a linker, to the C-terminus of one of the two light chains, and the other may be linked, either through a linker or directly without a linker, to the C-terminus of the other light chain of the anti-TIGIT antibody.

The linker may be a rigid peptide linker (e.g., [EAAAK]n, wherein n is an integer of 1 to 5), a flexible peptide linker (e.g., [(G)mS]l, wherein m is an integer of 1 to 5, and 1 is an integer of 1 to 5), but is not limited thereto.

### Anti-TIGIT Antibody or Antigen-Binding Fragment Thereof

The anti-TIGIT antibody or antigen-binding fragment thereof may include:
a polypeptide (CDR-H1) including the amino acid sequence of SEQ ID NO: 1, 2, or 3,
A polypeptide (CDR-H2) including the amino acid sequence of SEQ ID NO: 4, 5, or 6,
A polypeptide (CDR-H3) including the amino acid sequence of SEQ ID NO: 7, 8, or 9,
A polypeptide (CDR-L1) including the amino acid sequence of SEQ ID NO: 10, 13, or 14,
A polypeptide (CDR-L2) including the amino acid sequence of SEQ ID NO: 15, 16, or 17, and
A polypeptide (CDR-L3) including the amino acid sequence of SEQ ID NO: 18, 19, or 20.

The polypeptide (CDR-L1) including the amino acid sequence of SEQ ID NO: 10 may include the amino acid sequence of SEQ ID NO: 11 or SEQ ID NO: 12.

Herein, the complementarity-determining regions (CDRs) are determined based on the CDR definitions according to the Kabat system.

In an embodiment, six CDRs (CDR-L1, CDR-L2, CDR-L3, CDR-H1, CDR-H2, and CDR-H3) that may be included in the anti-TIGIT antibody or antigen-binding fragment thereof are summarized in Table 1 below:

**TABLE 1**

| | Sequence | SEQ ID NO |
|---|---|---|
| CDR-H1 | SDYAWN | 1 |
| CDR-H1 | GSTFTEYTMH | 2 |
| CDR-H1 | GYSFIDYIMN | 3 |
| CDR-H2 | YISYSGSARYNPSLKS | 4 |
| CDR-H2 | GLNPNNGGTSYNQRFKD | 5 |
| CDR-H2 | LSIPYNGGTSYNQKFEG | 6 |
| CDR-H3 | KGYPAYFAY | 7 |
| CDR-H3 | GTYYDYSFAY | 8 |
| CDR-H3 | GIKGYFAMDY | 9 |
| CDR-L1 | XASQDVSTAVA (X=K or R) | 10 |
| CDR-L1 | KASQDVSTAVA | 11 |
| CDR-L1 | RASQDVSTAVA | 12 |
| CDR-L1 | KASHYVSTAVA | 13 |
| CDR-L1 | RSSQSLVNSFGKTYLS | 14 |
| CDR-L2 | SASYRYT | 15 |
| CDR-L2 | SPSYRYT | 16 |
| CDR-L2 | GVSNRFS | 17 |
| CDR-L3 | QHHYSTPYT | 18 |
| CDR-L3 | HQHYSTPWT | 19 |
| CDR-L3 | LQGTHQPWT | 20 |

| | | |
|---|---|---|
| (In Table 1, CDR-H1, CDR-H2, and CDR-H3 represent complementarity-determining regions on the heavy chain, while CDR-L1, CDR-L2, and CDR-L3 represent complementarity-determining regions on the light chain.) | | |

In an embodiment, the anti-TIGIT antibody or antigen-binding fragment thereof may include:
a heavy chain variable region including CDR-H1 of SEQ ID NO: 1, 2, or 3, CDR-H2 of SEQ ID NO: 4, 5, or 6, and CDR-H3 of SEQ ID NO: 7, 8, or 9; and
a light chain variable region comprising CDR-L1 of SEQ ID NO: 10 (e.g., SEQ ID NO: 11 or 12), 13, or 14, CDR-L2 of SEQ ID NO: 15, 16, or 17, and CDR-L3 of SEQ ID NO: 18, 19, or 20.

More specifically, the anti-TIGIT antibody or antigen-binding fragment thereof may include:
a heavy chain variable region including the amino acid sequence of SEQ ID NO: 21, 22, 23, 24, 25, 26, 27, or 28; and
a light chain variable region including the amino acid sequence of SEQ ID NO: 29, 30, 31, 32, 33, 34, 35, or 36.

In some cases (e.g., when constructed recombinantly), the heavy chain variable region and/or light chain variable region may further include an appropriate signal sequence at the N-terminus thereof.

Table 2 below illustrates the amino acid sequences of heavy chain and light chain variable regions that may be included in the anti-TIGIT antibody or antigen-binding fragment thereof:

**TABLE 2**

| Variable Region | Amino Acid Sequence(N-C) | SEQ ID NO |
|---|---|---|
| Heavy chain variable region | DVQLQESGPGLVKPSQSLSLTCTVTGYSITSDYAWNWIRQFP GNKLEWMGYISYSGSARYNPSLKSRISITRDTSMNQFFLQLNS [?] VTAEDTATYYCARKGYPAYFAYWGQGTLVTVSS | 21 |
| Heavy chain variable region | DVQLQESGPGLVKPSQTLSLTCTVTGYSITSDYAWNWIRQPP GKGLEWMGYISYSGSARYNPSLKSRITISRDTSMNQFSLKLNS [?] VTAEDTATYYCARKGYPAYFAYWGQGTLVTVSS | 22 |
| Heavy chain variable region | DVQLQESGPGLVKPSQTLSLTCTVTGYSITSDYAWNWIRQPP GKGLEWMGYISYSGSARYNPSLKSRITISRDTSKNQFSLKLSS VTAEDTATYYCARKGYPAYFAYWGQGTLVTVSS | 23 |
| Heavy chain variable region | QVQLQESGPGLVKPSQTLSLTCTVTGYSITSDYAWNWIRQPP GKGLEWMGYISYSGSARYNPSLKSRVTISRDTSKNQFSLKLSS[?] VTAEDTATYYCARKGYPAYFAYWGQGTLVTVSS | 24 |
| Heavy chain variable region | QVQLQESGPGLVKPSQTLSLTCTVTGYSITSDYAWNWIRQPP GKGLEWMGYISYSGSARYNPSLKSRVTISRDTSKNQFSLKLSS[?] VTAADTAVYYCARKGYPAYFAYWGQGTLVTVSS | 25 |
| Heavy chain variable region | QVQLQESGPGLVKPSQTLSLTCTVTGYSITSDYAWNWIRQPP GKGLEWMGYISYSGSARYNPSLKSRVTISVDTSKNQFSLKLSS[?] VTAADTAVYYCARKGYPAYFAYWGQGTLVTVSS | 26 |
| Heavy chain variable region | EVQLQQSGPELVKPGASVKISCKTSGSTFTEYTMHWVKQSH GKSLEWIGGLNPNNGGTSYNQRFKDRATLTVDKSSSTAYME [?] LRSLTSEDSAVYYCTRGTYYDYSFAYWGQGTLVTVSA | 27 |
| Heavy chain variable region | EVQLQQSGPELVKPGASMKISCKASGYSFTDYIMNWVKQSH GKNLEWIGLSIPYNGGTSYNQKFEGKATLTVDKSSSTAYMEL LSLTSEDSAVYYCARGIKGYFAMDYWGQGTSVTVSS | 28 |
| Light chain variable region | DIVMTQSHKFMSTSVGDRVSISCKASQDVSTAVAWYQQKPG QSPELLIYSASYRYTGVPDRFTGSGSGTDFTFTISSVQAEDLAV YYCQHHYSTPYTFGGGTKLEMK | 29 |
| Light chain variable region | DIVMTQSHSFLSASVGDRVSITCKASQDVSTAVAWYQQKPG QAPELLIYSASYRYTGVPDRFTGSGSGTDFTLTISSLQAEDVA VYYCQHHYSTPYTFGQGTKLEMK | 30 |
| Light chain variable region | DIVMTQSPSSLSASVGDRVSITCKASQDVSTAVAWYQQKPGQ APRLLIYSASYRYTGVPDRFTGSGSGTDFTLTISSLQAEDVAV YYCQHHYSTPYTFGQGTKLEIK | 31 |
| Light chain variable region | DIQMTQSPSSLSASVGDRVSITCKASQDVSTAVAWYQQKPGQ APRLLIYSASYRYTGVPDRFSGSGSGTDFTLTISSLQAEDVAV YYCQHHYSTPYTFGQGTKLEIK | 32 |
| Light chain variable region | DIQMTQSPSSLSASVGDRVTITCRASQDVSTAVAWYQQKPGQ APRLLIYSASYRYTGVPDRFSGSGSGTDFTLTISSLQAEDVAV YYCQHHYSTPYTFGQGTKLEIK | 33 |
| Light chain variable region | DIQMTQSPSSLSASVGDRVSITCKASQDVSTAVAWYQQKPGQ APRLLIYSASYRYTGVPDRFSGSGSGTDFTLTISSLQPEDFATY YCQHHYSTPYTFGQGTKLEIK | 34 |
| Light chain variable region | DIVMTQSHKFMSTSVGDRVSITCKASHYVSTAVAWYQQKPG QSPKLLIYSPSYRYTGVPDRFTGSGSGTDFTFTISSVQAEDLAV YYCHQHYSTPWTFGGGTKLEIK | 35 |
| Light chain variable region | DVVVTQTPLSLPVSFGDQVSISCRSSQSLVNSFGKTYLSWFLH KPGQSPQLIMYGVSNRFSGVPDRFSGSGSGTDFTLKISTIKPED LGMYYCLQGTHQPWTFGGGTKLEIK | 36 |

| | | |
|---|---|---|
| (In Table 2, the underlined regions sequentially represent the CDR1, CDR2, and CDR3 of the heavy and light chains.) | | |

In an embodiment, the anti-TIGIT antibody or antigen-binding fragment thereof provided herein may bind to one or more amino acids selected from residues 51-70 (TAQVTQVNWEQQDQLLAICN; SEQ ID NO: 37) of the human TIGIT protein, for example, the human TIGIT protein (SEQ ID NO: 64; NCBI Reference Sequence NP_776160.2; UniProtKB/SwissProt Q495A1-1), but with no limitations thereto.

### [Human TIGIT protein (UniProtKB/SwissProt Q495A1-1)]

1 MRWCLLLIWA QGLRQAPLAS GMMTGTIETT GNISAEKGGS IILQCHLSST
TAQVTQVNWE
61 QQDQLLAICN ADLGWHISPS FKDRVAPGPG LGLTLQSLTV
NDTGEYFCIY HTYPDGTYTG
121 RIFLEVLESS VAEHGARFQI PLLGAMAATL VVICTAVIVV
VALTRKKKAL RIHSVEGDLR
181 RKSAGQEEWS PSAPSPPGSC VQAEAAPAGL CGEQRGEDCA
ELHDYFNVLS YRSLGNCSFF
241 TETG

As used herein, the term "antibody" refers to a protein that specifically binds to a particular antigen. This protein can be produced in response to antigen stimulation within the immune system or can be chemically synthesized or recombinantly produced, with no limitations to the types thereof. The antibody may be one that is non-naturally produced, for example, recombinantly or synthetically generated. The antibody may be an animal antibody (e.g., a mouse antibody), a chimeric antibody, a humanized antibody, or a human antibody. The antibody may be a monoclonal or polyclonal antibody.

In the anti-TIGIT antibody or antigen-binding fragment thereof provided herein, the regions other than the defined heavy chain CDR and light chain CDR regions, or the heavy chain variable region and light chain variable region, may be derived from immunoglobulins of any subtype (e.g., IgA, IgD, IgE, IgG (IgG1, IgG2, IgG3, or IgG4), IgM, etc.). For example, the remaining regions may be derived from the framework regions of any immunoglobulin subtype and/or from the constant regions of the light and/or heavy chains. In an embodiment, the anti-TIGIT antibody provided herein may be a human IgG-type antibody, for example, in the form of IgG1, IgG2, IgG3, or IgG4, but with no limitations thereto.

An intact antibody (e.g., IgG type) has a structure comprising two full-length light chains and two full-length heavy chains, with each light chain linked to a heavy chain via a disulfide bond. The constant region of the antibody is divided into a heavy chain constant region and a light chain constant region. The heavy chain constant region has gamma (γ), mu (µ), alpha (α), delta (δ), and epsilon (ε) types, with subclasses including gamma 1 (γ1), gamma 2 (γ2), gamma 3 (γ3), gamma 4 (γ4), alpha1 (α1), and alpha 2 (α2). The light chain constant region has kappa (κ) and lambda (λ) types.

In an embodiment, the anti-TIGIT antibody provided herein may include an IgG constant region as the heavy chain constant region and a kappa constant region as the light chain constant region, but is not limited thereto.

In an embodiment, the constant region of the IgG (e.g., human IgG1) may be wild-type. In another embodiment, the constant region of the IgG may be a variant including one or more mutations selected from the group consisting of S240D (substitution of serine at position 240 with aspartic acid), A331L, I333E, N298A, S299A, E334A, K335A, L235A, L236A, P330G, and K478 deletion (ΔK478), and may include, for example, the following mutations:
(1) S240D, A331L, and I333E;
(2) N298A;
(3) S299A, E334A, and K335A;
(4) L235A, L236A, and P330G; or
(5) K478 deletion.

The term "heavy chain" is construed to encompass both a full-length heavy chain and a fragment thereof that includes a variable region domain (V_{H}) containing an amino acid sequence sufficient to impart antigen specificity, and three constant region domains C_{H1}, C_{H2}, and C_{H3}, along with a hinge. The term "light chain" is construed to encompass both a full-length light chain and a fragment thereof that includes a variable region domain V_{L} containing an amino acid sequence sufficient to impart antigen specificity, and a constant region domain C_{L}.

The term "complementarity determining region" (CDR)" refers to a region of an antibody that confers antigen-binding specificity, comprising the amino acid sequences in the hypervariable regions of the heavy and light chains of immunoglobulins. The heavy chain and light chain each may contain three CDRs (CDRH1, CDRH2, CDRH3, and CDRL1, CDRL2, CDRL3). These CDRs may provide key contact residues for the antibody's binding to an antigen or epitope. As used herein, the term "specifically binds" or "specifically recognizes" refers to the interaction between an antigen and antibody that results in an immunological response, as is conventionally known in the art.

The complementarity-determining regions (CDRs) described herein are defined based on the Kabat system.

Unless otherwise specified, the term "antibody", as used herein, can be understood to encompass antigen-binding fragments of antibodies that retain antigen-binding ability.

The term "antigen-binding fragment" refers to any form of polypeptide that includes a portion capable of binding to an antigen (e.g., the six CDRs defined herein). Examples include scFv, scFv-Fc, (scFv)2, Fab, Fab', or F(ab')2, but are not limited thereto.

Among the antigen-binding fragments, Fab has a structure that includes the variable regions of the light and heavy chains, the constant region of the light chain, and the first constant region (C_{H1}) of the heavy chain, having a single antigen-binding site.

Fab' differs from Fab in that the former includes a hinge region containing one or more cysteine residues at the C-terminus of the C_{H1} domain of the heavy chain.

F(ab')2 antibodies are produced when the cysteine residues in the hinge region of Fab' form disulfide bonds. Fv is the smallest antibody fragment, including only the variable regions of the heavy and light chains, and recombinant techniques for generating Fv fragments are well known in the art.

Two-chain Fv includes a variable region of the heavy chain and a variable region of the light chain, with a non-covalent linkage therebetween, while single-chain Fv (scFv) includes a variable region of the light chain and a variable region of the heavy chain, which may be covalently bonded to each other generally via a peptide linker or directly at the C-terminus, forming a dimer-like structure similar to two-chain Fv.

The antigen-binding fragment may be obtained using proteolytic enzymes (e.g., Fab can be obtained by restrictive digestion of the whole antibody with papain, while F(ab')2 fragments can be obtained by digestion with pepsin) or can be produced through recombinant DNA technology.

The term "hinge region" refers to a region included in the heavy chain of an antibody, located between the C_{H1} and C_{H2} domains, which functions to provide flexibility to the antigen-binding site within the antibody.

The antibody provided herein may be a monoclonal antibody. Monoclonal antibodies may be produced by methods well known in the art. For example, they can be produced using phage display techniques or can be generated as monoclonal antibodies derived from animals (e.g., mice) using conventional methods.

Individual monoclonal antibodies may be screened based on their ability to bind to the receptor-binding domain of TIGIT using standard ELISA (Enzyme-Linked ImmunoSorbent Assay) formats. Functional assays such as competitive ELISA or cell-based assays can be used to assess molecular interactions and inhibitory activity. The affinity (Kd values) of each monoclonal antibody for the receptor-binding domain of TIGIT can then be evaluated based on the selected antibodies with strong inhibitory activity.

In the final selected antibodies, the regions other than the antigen-binding site can be humanized or human immunoglobulin antibodies, and methods for producing humanized antibodies are well known in the art.

The antigen-binding fragments of the anti-TIGIT antibody provided herein refer to fragments derived from the anti-TIGIT antibody that retain binding affinity for the antigen (TIGIT), and may be any polypeptide comprising the six CDRs of the anti-TIGIT antibody described above, such as scFv, scFv-Fc, scFv-Ck (kappa constant region), scFv-Cλ (lambda constant region), (scFv)2, Fab, Fab', or F(ab')₂, but with no limitations thereto. In an embodiment, the antigen-binding fragment may be scFv, or a fusion polypeptide in which scFv is fused to the Fc region of an immunoglobulin (e.g., IgG1, IgG2, IgG3, IgG4, etc.) (scFv-Fc) or to the constant region of a light chain (e.g., kappa or lambda) (scFv-Ck or scFv-Cλ), but is not limited thereto.

As used herein, the wording that an antibody (e.g., CDR, variable region, or heavy/light chain, antigen-binding fragment, etc.) "includes a specific amino acid sequence", or "comprises or consists of a specific amino acid sequence" covers not only the case where the antibody necessarily includes the specific amino acid sequence, but also the case where modifications (e.g., substitutions, deletions, and/or additions of amino acid residues) that do not significantly affect the antibody's activity (e.g., antigen affinity, pharmacological activity, etc.) are introduced into the amino acid sequence.

The anti-TIGIT antibody or antigen-binding fragment provided herein may have a binding affinity (K_{D}) for TIGIT (e.g., human TIGIT) of, for example, for example, 10 mM or lower, 5 mM or lower, 1 mM or lower, 0.5 mM or lower, 0.2 mM or lower, 0.1 mM or lower, 0.05 mM or lower, 0.01 mM or lower, 0.005 mM or lower, or 0.001 mM or lower, as measured by surface plasmon resonance (SPR). Specifically, the K_{D} may be within a range of 0.0001 nM to 10 mM, 0.0005 nM to 10 mM, 0.001 nM to 10 mM, 0.005 nM to 10 mM, 0.01 nM to 10 mM, 0.05 nM to 10 mM, 0.1 nM to 10 mM, 0.5 nM to 10 mM, 1 nM to 10 mM, 0.0001 nM to 5 mM, 0.0005 nM to 5 mM, 0.001 nM to 5 mM, 0.005 nM to 5 mM, 0.01 nM to 5 mM, 0.05 nM to 5 mM, 0.1 nM to 5 mM, 0.5 nM to 5 mM, 1 nM to 5 mM, 0.0001 nM to 1 mM, 0.0005 nM to 1 mM, 0.001 nM to 1 mM, 0.005 nM to 1 mM, 0.01 nM to 1 mM, 0.05 nM to 1 mM, 0.1 nM to 1 mM, 0.5 nM to 1 mM, 1 nM to 1 mM, 0.0001 nM to 0.5 mM, 0.0005 nM to 0.5 mM, 0.001 nM to 0.5 mM, 0.005 nM to 0.5 mM, 0.01 nM to 0.5 mM, 0.05 nM to 0.5 mM, 0.1 nM to 0.5 mM, 0.5 nM to 0.5 mM, 1 nM to 0.5 mM, 0.0001 nM to 0.2 mM, 0.0005 nM to 0.2 mM, 0.001 nM to 0.2 mM, 0.005 nM to 0.2 mM, 0.01 nM to 0.2 mM, 0.05 nM to 0.2 mM, 0.1 nM to 0.2 mM, 0.5 nM to 0.2 mM, 1 nM to 0.2 mM, 0.0001 nM to 0.1 mM, 0.0005 nM to 0.1 mM, 0.001 nM to 0.1 mM, 0.005 nM to 0.1 mM, 0.01 nM to 0.1 mM, 0.05 nM to 0.1 mM, 0.1 nM to 0.1 mM, 0.5 nM to 0.1 mM, 1 nM to 0.1 mM, 0.0001 nM to 0.05 mM, 0.0005 nM to 0.05 mM, 0.001 nM to 0.05 mM, 0.005 nM to 0.05 mM, 0.01 nM to 0.05 mM, 0.05 nM to 0.05 mM, 0.1 nM to 0.05 mM, 0.5 nM to 0.05 mM, or 1 nM to 0.05 mM, but is not limited thereto.

### Interleukin-15 (IL-15)

The interleukin-15 (IL-15) may be selected from the group consisting of:
(1) a wild-type IL-15 polypeptide,
(2) a polypeptide having a homology of 60% or more, 70% or more, 80% or more, 85% or more, 90% or more, 91% or more, 92% or more, 93% or more, 94% or more, 95% or more, 96% or more, 97% or more, 98% or more, or 99% or more with the polypeptide (1).

Interleukin-15 (IL-15) has structural similarity to interleukin-2 (IL-2) and binds to the IL-2/IL-15 receptor beta chain (CD122) and the common gamma chain (gamma-C, CD132) complex, through which it participates in signal transduction. In an example, IL-15 may be derived from humans, for example, NCBI Accession No. NP_000576.1 (amino acid residues 49-162), but with no limitations thereto.

In an embodiment, the IL-15 may have the following amino acid sequence (N-terminus → C-terminus):

NWVNVISDLKKIEDLIQSMHIDATLYTESDVHPSCKVTAMKCFLLELQVISLE
SGDASIHDTVENLIILANNSLSSNGNVTESGCKECEELEEKNIKEFLQSFVHIVQMFIN
TS (SEQ ID NO: 38).

The polypeptide (1) may include an additional oligopeptide for additional purposes, for example, to facilitate protein purification, such as a tag for labeling at the N-terminus (e.g., MAPRRARGCRTLGLPALLLLLLLRPPATRGDYKDDDDKIEGR (SEQ ID NO: 76)), but with no limitations thereto.

The interleukin-15 is characterized by low immunogenicity when administered to the human body due to its human origin.

The IL-15 may exhibit binding activity to its receptor (e.g., binding activity to the IL-2/IL-15 receptor beta chain (CD122) and/or the IL-2/IL-15 receptor beta chain (CD122) and the common gamma chain (gamma-C, CD132) complex), and/or cytokine production and/or secretion activity in immune cells (e.g., T cells (CD4+, CD8+), NK cells, etc.).

In an embodiment, the IL-15 may be selected from the group consisting of:
(1) a polypeptide including the amino acid sequence of SEQ ID NO: 38,
(2) a polypeptide having a homology of 93% or more, 94% or more, 95% or more, 96% or more, 97% or more, 98% or more, or 99% or more to the polypeptide (1), and exhibiting binding activity to the IL-2/IL-15 receptor and/or cytokine production and/or secretion activity in immune cells. The IL-15 may exhibit superior binding activity to the IL-2/IL-15 receptor, and/or cytokine production and/or secretion activity in immune cells, and/or anticancer activity (e.g., inhibiting cancer cell proliferation, inducing cancer cell apoptosis).

### Linker

The linker may be a peptide linker, for example, [EAAAK]n (n is an integer from 1 to 5 representing the number of [EAAAK] (SEQ ID NO: 77)), [(G)mS]l (m is an integer from 1 to 5 representing the number of G residues, and 1 is an integer from 1 to 5 representing the number of [(G)mS]), A EAAAK A (SEQ ID NO: 78), A EAAAK EAAAK A (SEQ ID NO: 79), A EAAAK EAAAK EAAAK A (SEQ ID NO: 80), A EAAAK EAAAK EAAAK EAAAK A (SEQ ID NO: 81), A EAAAK EAAAK EAAAK EAAAK EAAAK A (SEQ ID NO: 82), AEAAAKEAAAKAG (SEQ ID NO: 83), AEAAAKEAAAKAGS (SEQ ID NO: 84), GGGGG (SEQ ID NO: 85), GGAGG (SEQ ID NO: 86), GGGGGGGG (SEQ ID NO: 87), GAGAGAGAGA (SEQ ID NO: 88), RPLSYRPPFPFGFPSVRP (SEQ ID NO: 89), YPRSIYIRRRHPSPSLTT (SEQ ID NO: 90), TPSHLSHILPSFGLPTFN (SEQ ID NO: 91), RPVSPFTFPRLSNSWLPA (SEQ ID NO: 92), SPAAHFPRSIPRPGPIRT (SEQ ID NO: 93), APGPSAPSHRSLPSRAFG (SEQ ID NO: 94), PRNSIHFLHPLLVAPLGA (SEQ ID NO: 95), MPSLSGVLQVRYLSPPDL (SEQ ID NO: 96), SPQYPSPLTLTLPPHPSL (SEQ ID NO: 97), NPSLNPPSYLHRAPSRIS (SEQ ID NO: 98), LPWRTSLLPSLPLRRRP (SEQ ID NO: 99), PPLFAKGPVGLLSRSFPP (SEQ ID NO: 100), VPPAPVVSLRSAHARPPY (SEQ ID NO: 101), LRPTPPRVRSYTCCPTP (SEQ ID NO: 102), PNVAHVLPLLTVPWDNLR (SEQ ID NO: 103), CNPLLPLCARSPAVRTFP (SEQ ID NO: 104), LGTPTPTPTPTGEF (SEQ ID NO: 105), EDFTRGKL (SEQ ID NO: 106), L EAAAR EAAAR EAAAR EAAAR (SEQ ID NO: 107), L EAAAR EAAAR EAAAR (SEQ ID NO: 108), L EAAAR (SEQ ID NO: 109), L EAAAR EAAAR (SEQ ID NO: 110), EAAAR EAAAR EAAAR EAAAR (SEQ ID NO: 111), EAAAR EAAAR EAAAR (SEQ ID NO: 112), EAAAR EAAAR (SEQ ID NO: 113), EAAAR (SEQ ID NO: 114), LTEEQQEGGG (SEQ ID NO: 115), TEEQQEGGG (SEQ ID NO: 116), LAKLKQKTEQLQDRIAGGG (SEQ ID NO: 117), LELKTPLGDT (SEQ ID NO: 118), THTCPRCPEP (SEQ ID NO: 119), KSCDTPPPCP (SEQ ID NO: 120), RCPEPKSCDT (SEQ ID NO: 121), PPPCPRCPEP (SEQ ID NO: 122), KSCDTPPPCP (SEQ ID NO: 123), RCPGG (SEQ ID NO: 124), and LEPKSSDKTHTSPPSPGG (SEQ ID NO: 125), but are not limited thereto. In an embodiment, the peptide linker may be a rigid linker, such as [EAAAK]n (where n is an integer from 1 to 5 representing the number of [EAAAK]), or a flexible linker, such as [(G)mS]l (where m is an integer from 1 to 5 representing the number of G residues, and 1 is an integer from 1 to 5 representing the number of [(G)mS]), but is not limited thereto.

### Nucleic Acid Molecules, Recombinant Vectors, and Recombinant Cells

Another aspect provides a nucleic acid molecule encoding the fusion protein.

Another aspect provides a recombinant vector carrying the nucleic acid molecule. The vector may be used as an expression vector for the nucleic acid molecule.

Another aspect provides a recombinant cell anchoring the nucleic acid molecule or a recombinant vector carrying same. The cell may be used for the production of the fusion protein.

Another aspect provides a method for producing the fusion protein, the method comprising a step of expressing the nucleic acid molecule in an appropriate host cell. The step of expressing the nucleic acid molecule in an appropriate host cell may include culturing the recombinant cell described above under a standard condition.

The term "vector" refers to a means for expressing a target gene in a host cell. Examples of the vector include plasmid vectors, cosmid vectors, and viral vectors such as bacteriophage vectors, adenovirus vectors, retrovirus vectors, and adeno-associated virus vectors. Vectors that may be used as recombinant vectors include commonly used plasmids in the art (e.g., pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19), phages (e.g., λgt4λB, λ-Charon, λΔz1, and M13), or viruses (e.g., SV40).

In the recombinant vector, the nucleic acid molecule encoding the fusion protein may be operatively linked to a promoter. The term "operatively linked" refers to a functional connection between a nucleotide regulatory sequence (e.g., a promoter sequence) and another nucleotide sequence. The regulatory sequence, when "operatively linked", controls the transcription and/or translation of the other nucleotide sequence.

The recombinant vector may typically be constructed as a cloning vector or an expression vector. The expression vector may be any conventional vector used in the art for expressing foreign proteins in plants, animals, or microorganisms. The recombinant vector may be constructed using various methods known in the art.

The recombinant vector may be constructed using prokaryotic or eukaryotic cells as a host. For example, when the vector is an expression vector and prokaryotic cells are used as the host, it is common for the vector to include a strong promoter capable of driving transcription (e.g., pL^{λ} promoter, CMV promoter, *trp* promoter, *lac* promoter, *tac* promoter, T7 promoter, etc.), a ribosome binding site for initiating translation, and transcription/translation termination sequences. When eukaryotic cells are used as the host, the vector may include eukaryotic cell-origin replication origins such as f1 origin, SV40 origin, pMB1 origin, adenovirus origin, AAV origin, and BBV origin, but is not limited thereto. Additionally, the vector may include a promoter derived from a mammalian genome (e.g., metallothionein promoter) or from a mammalian virus (e.g., adenovirus late promoter, vaccinia virus 7.5K promoter, SV40 promoter, cytomegalovirus promoter, and HSV *tk* promoter), and typically includes a polyadenylation sequence as a transcription termination sequence.

The recombinant cell may be obtained by introducing the recombinant vector into an appropriate host cell. So long as it is known in the art, any host cell that can stably and continuously clone or express the recombinant vector may be available. Prokaryotic host cells may include, for example, *E*. *coli* JM109, *E*. *coli* BL21, *E*. *coli* RR1, *E*. *coli* LE392, *E*. *coli* B, *E. coli* X 1776, *E. coli* W3110, *Bacillus* spp. such as *Bacillus subtilis* and *Bacillus thuringiensis,* and gut bacteria such as *Salmonella typhimurium, Serratia marcescens,* and various *Pseudomonas* species. For eukaryotic host cells, transformed host cells may include yeast (*Saccharomyces cerevisiae*)*,* insect cells, plant cells, and animal cells, for example, Sp2/0, CHO (Chinese hamster ovary) K1, CHO, DG44, PER.C6, W138, BHK, COS-7, 293, HepG2, Huh7, 3T3, RIN, MDCK cell lines, but are not limited thereto.

The delivery (introduction) of the nucleic acid molecule or recombinant vector into the host cell may be performed using commonly known methods. For example, when the host cell is prokaryotic, methods such as the CaCl₂ method or electroporation may be used, and when the host cell is eukaryotic, methods such as microinjection, calcium phosphate precipitation, electroporation, liposome-mediated transfection, or gene bombardment may be used, but with no limitations thereto.

The method for selecting the transformed (recombinant vector-introduced) host cells can be easily performed using well-known methods in the art based on the phenotype expressed by a selectable marker. For example, if the selectable marker is a gene conferring resistance to a specific antibiotic, recombinant cells into which the recombinant vector has been introduced can be easily selected by culturing in a medium containing the antibiotic.

### Pharmaceutical Uses

The anti-TIGIT antibody or antigen-binding fragment thereof included in the fusion protein provided herein functions to block the action of TIGIT (e.g., the interaction between TIGIT and its ligand CD155 (PVR)), thereby activating the immune system (e.g., enhancing effector T cell function, regulating Treg activity, increasing cytokine secretion), and also exhibits excellent anticancer effects (e.g., cancer cell apoptosis, inhibition of cancer cell proliferation). Additionally, IL-15 has high binding affinity to its receptor (IL-2/IL-15 receptor) and low immunogenicity, resulting in enhanced immune responses such as increased cytokine secretion when administered in vivo, while maintaining safety by minimizing the risk of being recognized as an immunogen. IL-15 also exhibits excellent anticancer effects (e.g., cancer cell apoptosis, inhibition of cancer cell proliferation). Therefore, the fusion peptide combines the advantages of the anti-TIGIT antibody or its antigen-binding fragment and IL-15, and can be effectively applied for immune enhancement, prevention and/or treatment of immune-related diseases, particularly for cancer prevention and/or treatment.

An aspect provides an immune enhancer or pharmaceutical composition for immune enhancement, comprising, as an active ingredient, at least one selected from the group consisting of the fusion protein, a nucleic acid molecule encoding the fusion protein, a recombinant vector carrying the nucleic acid molecule, and a recombinant cell anchoring the nucleic acid molecule or recombinant vector.

Another aspect provides a pharmaceutical composition for the prevention and/or treatment of immune-related diseases, the composition including, as an active ingredient, at least one selected from the group consisting of the fusion protein, a nucleic acid molecule encoding the fusion protein, a recombinant vector carrying the nucleic acid molecule, and a recombinant cell anchoring the nucleic acid molecule or recombinant vector.

Another aspect provides an anticancer agent or pharmaceutical composition for the prevention and/or treatment of cancer, comprising, as an active ingredient, at least one selected from the group consisting of the fusion protein, a nucleic acid molecule encoding the fusion protein, a recombinant vector carrying the nucleic acid molecule, and a recombinant cell anchoring the nucleic acid molecule or recombinant vector.

Another aspect provides a method for enhancing immunity, the method comprising a step of administering a pharmaceutically effective amount of at least one selected from the group consisting of the fusion protein, a nucleic acid molecule encoding the fusion protein, a recombinant vector carrying the nucleic acid molecule, and a recombinant cell anchoring the nucleic acid molecule or recombinant vector to a subject in need of immune enhancement. The immune enhancement method may further include the step of identifying the subject in need of immune enhancement prior to the administration step.

Another aspect provides a method for the prevention and/or treatment of immune-related diseases, the method comprising the step of administering a pharmaceutically effective amount of at least one selected from the group consisting of the fusion protein, a nucleic acid molecule encoding the fusion protein, a recombinant vector carrying the nucleic acid molecule, and a recombinant cell anchoring the nucleic acid molecule or recombinant vector to a subject in need of prevention and/or treatment of immune-related diseases. The method for the prevention and/or treatment of immune-related diseases may further include a step of identifying the subject in need of prevention and/or treatment of immune-related diseases prior to the administration step.

Another aspect provides a method for the prevention and/or treatment of cancer, the method comprising a step of administering a pharmaceutically effective amount of at least one selected from the group consisting of the fusion protein, a nucleic acid molecule encoding the fusion protein, a recombinant vector carrying the nucleic acid molecule, and a recombinant cell anchoring the nucleic acid molecule or recombinant vector to a subject in need of prevention and/or treatment of cancer. The method for the prevention and/or treatment of cancer may further include the step of identifying the subject in need of prevention and/or treatment of cancer prior to the administration step.

Another aspect provides the use of at least one selected from the group consisting of the fusion protein, a nucleic acid molecule encoding the fusion protein, a recombinant vector carrying the nucleic acid molecule, and a recombinant cell anchoring the nucleic acid molecule or recombinant vector for immune enhancement, prevention and/or treatment of immune-related diseases, and/or prevention and/or treatment of cancer, or for the manufacture of a pharmaceutical composition for immune enhancement, prevention and/or treatment of immune-related diseases, and/or prevention and/or treatment of cancer.

As used herein, the term "immune enhancement" refers to inducing an initial immune response to an antigen or increasing an existing immune response, and can be used interchangeably with terms such as immunopotentiation, immune activation, and the like. More specifically, the "immune-enhancing effect" of the fusion protein provided herein may include, but is not limited to, enhanced function (activation) and/or proliferation of immune cells (e.g., NK cells, NKT cells, CD3+ T cells, effector T cells (CD4+ T cells), cytotoxic T cells (CD8+ T cells), etc.), activity inhibition and/or depletion of regulatory T (Treg) cells, increased production and/or secretion of immune proteins (e.g., cytokines such as IL-2, IFN-gamma, etc.), and activation (e.g., confirmed as IL2RB/IL2RG dimerization, etc.) of IL-15 receptors (located on the surface of immune cells (NK cells, etc.)).

As used herein, the term "immune-related disease" is intended to encompass all diseases caused by immune system disorders and/or insufficient activation, including, but not limited to cancer, infectious diseases, autoimmune diseases, and inflammatory diseases.

The cancer may be a solid tumor or blood cancer, and may be at least one selected from the group consisting of squamous cell carcinoma, lung cancer (e.g., small cell lung cancer, non-small cell lung cancer, lung adenocarcinoma, lung squamous cell carcinoma), peritoneal cancer, skin cancer, melanoma (e.g., cutaneous or ocular melanoma), rectal cancer, esophageal cancer, small intestine cancer, endocrine gland cancer, thyroid cancer, parathyroid cancer, adrenal cancer, soft tissue sarcoma, urethral cancer, chronic or acute leukemia, lymphocytic lymphoma, liver cancer, gastric cancer, pancreatic cancer, glioblastoma, cervical cancer, ovarian cancer, bladder cancer, breast cancer, colon cancer, colorectal cancer, endometrial cancer or uterine cancer, salivary gland cancer, kidney cancer, prostate cancer, vulvar cancer, head and neck cancer, brain cancer, and osteosarcoma, but with no limitations thereto.

The prophylactic and/or therapeutic effects of cancer include not only inhibiting the occurrence and/or growth of cancer cells but also suppressing cancer progression caused by migration, invasion, and metastasis.

The infectious diseases, autoimmune diseases, and inflammatory diseases may be any disease that can be treated, alleviated, and/or prevented through the immune enhancement described in the foregoing (for example, enhanced function (activation) and/or proliferation of immune cells (e.g., NK cells, NKT cells, CD3+ T cells, effector T cells (CD4+ T cells), cytotoxic T cells (CD8+ T cells), etc.), activity inhibition and/or depletion of regulatory T (Treg) cells, increased production and/or secretion of immune proteins (e.g., cytokines such as IL-2, IFN-gamma, etc.), etc.). For example, the infectious disease is a generic name for diseases caused by pathogens such as viruses (for example, influenza virus, coronavirus (e.g., Severe Acute Respiratory Syndrome (SARS) virus (SARS-CoV, SARS-CoV-2), Middle East Respiratory Syndrome (MERS) virus (MERS-CoV), cytomegalovirus (CMV), Epstein-Barr virus (EBV)), bacteria, fungi, or parasites that spread and invade organisms (e.g., animals, including humans) and may include infections caused by at least one pathogen selected from the group consisting of viruses, bacteria, fungi, and parasites, and the resulting diseases. Autoimmune diseases may include, but are not limited to, rheumatoid arthritis, type 1 diabetes, Crohn's disease, ulcerative colitis, Behcet's syndrome, lupus, Sjogren's syndrome, myasthenia gravis, scleroderma, hypothyroidism, hyperthyroidism, psoriasis, vitiligo, multiple sclerosis, autoimmune hepatitis, autoimmune nephritis, autoimmune pancreatitis, autoimmune encephalitis, and cytokine storm. The inflammatory disease is a generic name for inflammations (chronic or acute inflammation) or diseases caused by inflammation, and may be at least one selected from the group consisting of heart inflammation (e.g., coronary artery disease, angina, myocardial infarction, pericarditis, myocarditis), vascular inflammation (e.g., atherosclerosis, vasculitis, disseminated intravascular coagulation (DIC), immune thrombocytopenic purpura (ITP), thrombotic thrombocytopenic purpura (TTP), anemia), upper respiratory tract inflammation (e.g., acute nasopharyngitis, allergic rhinitis, sinusitis, pharyngitis, tonsillitis, laryngitis), lower respiratory tract and/or lung inflammation (e.g., bronchitis, bronchiectasis, asthma, chronic obstructive pulmonary disease (COPD), pneumonia, interstitial lung disease, tuberculosis), upper gastrointestinal inflammation (e.g., gastritis, esophagitis), lower gastrointestinal inflammation (e.g., enteritis, ulcerative colitis, Crohn's disease, celiac disease, diverticulitis, irritable bowel syndrome, appendicitis, fistula), liver, bile duct, and/or pancreatic inflammation (e.g., hepatitis, fatty liver, cholangitis, cholecystitis, pancreatitis, type 1 diabetes), renal (upper urinary tract) inflammation (e.g., pyelonephritis, glomerulonephritis, urinary tract infections), lower urinary tract inflammation (e.g., urinary tract infections, ureteritis, urethritis, cystitis, prostatitis/chronic pelvic pain syndrome), thyroid and/or parathyroid inflammation (e.g., thyroiditis, parathyroiditis), adrenal inflammation (e.g., adrenalitis), reproductive organ inflammation (e.g., pelvic inflammatory disease, oophoritis, orchitis, epididymitis), bone and/or joint inflammation (e.g., osteoarthritis, rheumatoid arthritis, osteomyelitis, synovitis), skin inflammation (e.g., cellulitis, erysipelas, tinea, athlete's foot, acne), muscle inflammation (e.g., myositis), brain inflammation (e.g., encephalitis, major depressive disorder), nerve inflammation (e.g., neuritis in various locations such as eyes or ears, complex regional pain syndrome, Guillain-Barré syndrome), eye inflammation (e.g., stye, uveitis, conjunctivitis), ear inflammation (e.g., otitis media, mastoiditis), oral inflammation (e.g., stomatitis, periodontitis, gingivitis), systemic inflammation (e.g., systemic inflammatory response syndrome (SIRS), metabolic syndrome-related diseases), peritonitis, reperfusion injury, transplant rejection, and hypersensitivity reactions, but with no limitations thereto.

The subject to which the fusion protein and/or pharmaceutical composition provided herein may be administered may be any animal or cell. For example, the subject may be an animal selected from mammals, including humans, primates such as monkeys, rodents such as rats and mice, or cells, tissues, body fluids (e.g., serum), or cultures derived (isolated) from these animals, such as human cells, tissues, or body fluids (e.g., serum) derived from humans.

The pharmaceutical composition may additionally include a pharmaceutically acceptable carrier in addition to the fusion protein, nucleic acid molecule, vector, and/or cell as the active ingredient. The carrier may be any one commonly used for formulating protein drugs and may be selected from the group consisting of lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylparaben, propylparaben, talc, magnesium stearate, and mineral oil, but with no limitations thereto. The pharmaceutical composition may further include at least one selected from the group consisting of diluents, excipients, lubricants, wetting agents, sweeteners, flavoring agents, emulsifiers, suspending agents, and preservatives commonly used in the preparation of pharmaceutical compositions.

The content of the fusion protein, nucleic acid molecule, vector, and/or cell as the active ingredient in the pharmaceutical composition may range from 0.00001 to 99 wt%, 0.0001 to 99 wt%, 0.001 to 99 wt%, 0.01 to 99 wt%, 0.1 to 99 wt%, 1 to 99 wt%, 10 to 99 wt%, 15 to 99 wt%, 20 to 99 wt%, 25 to 99 wt%, 30 to 99 wt%, 35 to 99 wt%, 40 to 99 wt%, 45 to 99 wt%, or 50 to 99 wt%, based on the total weight of the composition, but is not limited thereto.

The administration of the fusion protein, nucleic acid molecule, vector, cells, and/or pharmaceutical composition may be via oral or non-oral routes. Non-oral administration may include intravenous injection, subcutaneous injection, intramuscular injection, intraperitoneal injection, intradermal injection, topical application, nasal administration, pulmonary administration, and rectal administration. For oral administration, since proteins or peptides are digested, oral compositions should be formulated to protect the active agent from degradation in the stomach.

Additionally, the fusion protein, nucleic acid molecule, vector, cell, and/or pharmaceutical composition may be formulated as a solution, suspension, syrup, or emulsion in an oil or aqueous medium, or as an extract, powder, granule, tablet, capsule, or injection, and may further include dispersants or stabilizers for formulation.

The content of the active ingredient in the pharmaceutical composition may vary depending on the method of formulation, route of administration, age, weight, sex, disease state of the patient, diet, time of administration, dosing interval, route of administration, rate of excretion, and response sensitivity. The daily dose of the pharmaceutical composition, based on the active ingredient (fusion protein, nucleic acid molecule, vector, cell), may range from 0.00001 to 1000 mg/kg, 0.00001 to 500 mg/kg, 0.00001 to 100 mg/kg, 0.00001 to 50 mg/kg, 0.0001 to 1000 mg/kg, 0.0001 to 500 mg/kg, 0.0001 to 100 mg/kg, 0.0001 to 50 mg/kg, 0.001 to 1000 mg/kg, 0.001 to 500 mg/kg, 0.001 to 100 mg/kg, 0.001 to 50 mg/kg, 0.01 to 1000 mg/kg, 0.01 to 500 mg/kg, 0.01 to 100 mg/kg, 0.01 to 50 mg/kg, 0.1 to 1000 mg/kg, 0.1 to 500 mg/kg, 0.1 to 100 mg/kg, or 0.1 to 50 mg/kg, but is not limited thereto. The daily dose may be formulated as a single unit dose or divided appropriately, or manufactured as a multi-dose container. As used herein, the term "pharmaceutically effective amount" refers to an amount of the active ingredient that exhibits the desired pharmaceutical activity, which may fall within the aforementioned dosage ranges.

Another aspect provides a health functional food for immune enhancement or metabolic promotion, the composition including, as an active ingredient, at least one selected from the group consisting of the fusion protein, a nucleic acid molecule encoding the fusion protein, a recombinant vector carrying the nucleic acid molecule, and a recombinant cell anchoring the nucleic acid molecule or recombinant vector.

Another aspect provides a health functional food for the prevention and/or improvement of immune-related diseases, the food including, as an active ingredient, at least one selected from the group consisting of the fusion protein, a nucleic acid molecule encoding the fusion protein, a recombinant vector carrying the nucleic acid molecule, and a recombinant cell anchoring the nucleic acid molecule or recombinant vector.

Another aspect provides a health functional food for the prevention and/or improvement of cancer, the food including, as an active ingredient, at least one selected from the group consisting of the fusion protein, a nucleic acid molecule encoding the fusion protein, a recombinant vector carrying the nucleic acid molecule, and a recombinant cell anchoring the nucleic acid molecule or recombinant vector.

The term "health functional food", as used herein, refers to a food product manufactured (processed) using nutrients that are easily deficient in daily diet, or raw materials or ingredients with useful functions for the human body (hereinafter referred to as "functional raw materials"), and is intended to encompass all foods that are beneficial for maintaining health or preventing and/or alleviating certain diseases or symptoms. No particular limitations are imparted to the final product. For example, the health functional food may be selected from the group consisting of various foods, beverages, and food additives, but with no limitations thereto.

The food composition may include acceptable food additives, and may further include appropriate carriers, fillers, and diluents commonly used in the production of health functional foods.

When used as a food additive, the food composition may be added directly or used in conjunction with other foods or food ingredients, and may be appropriately used by conventional methods.

The content of the active ingredient in the food composition may be determined appropriately depending on the form of the food, the desired effect, and the purpose, and may range from 0.00001 to 99 wt%, 0.0001 to 99 wt%, 0.001 to 99 wt%, 0.01 to 99 wt%, 0.1 to 99 wt%, 1 to 99 wt%, or 10 to 99 wt% of the total food weight, but is not limited thereto.

### Advantageous Effects

The anti-TIGIT antibody or antigen-binding fragment thereof included in the fusion protein provided herein blocks the action of TIGIT (e.g., the interaction between TIGIT and its ligand CD155 (PVR)), thereby activating the immune system (e.g., enhancing effector T cell function, regulating and/or depleting Treg cells, increasing cytokine secretion) and exhibiting excellent anticancer effects (e.g., cancer cell apoptosis, inhibition of cancer cell proliferation). Furthermore, IL-15 has high binding affinity to its receptor (IL-2/IL-15 receptor) and low immunogenicity, resulting in strong immune-enhancing effects, such as increased cytokine secretion when administered in vivo, while maintaining safety by minimizing the risk of triggering an immune response as an immunogen. IL-15 also exhibits excellent anticancer effects (e.g., cancer cell apoptosis, inhibition of cancer cell proliferation). Thus, the fusion peptide combines the benefits of the anti-TIGIT antibody or its antigen-binding fragment and IL-15, making it useful for immune enhancement, prevention and/or treatment of immune-related diseases, particularly for cancer prevention and/or treatment.

### Brief Description of the Drawings

FIG. 1 is a plot of the binding affinity of the fusion protein for human TIGIT protein according to an embodiment.
FIGS. 2a to 2c are graphs showing the increased cytokine production in immune cells by the fusion protein according to an embodiment (2a: CD4+ T cells, 2b: CD8+ T cells, 2c: CD56+ NK cells).
FIG. 3 shows graphs of increased cytokine expression in PBMCs by the fusion protein according to an embodiment.
FIGS. 4a and 4b are plots of STAT5 induction in immune cells by the fusion protein according to an embodiment (FIG. 4a: CD8+ T cells, FIG. 4b: NK cells).
FIG. 5 shows graphs of the long-term cytokine production effect in immune cells by the fusion protein according to an embodiment.
FIG. 6 is a graph showing the depletion capability of regulatory T cells by the fusion protein according to an embodiment.
FIGS. 7a to 7c are graphs of the proliferation capacity of immune cells by the fusion protein according to an embodiment (7a: T cells, 7b: NKT cells, 7c: NK cells).
FIG. 8 is a graph showing sustained cytokine production in NK cells by the fusion protein according to an embodiment.
FIG. 9 depicts the cytotoxicity of the fusion protein against lung cancer cells (A549 cells) according to an embodiment.
FIG. 10 depicts the cytotoxicity of the fusion protein against breast cancer cells (MCF-7 cells) according to an embodiment.

### Mode for Invention

A better understanding of the present disclosure may be obtained through the following examples, which are set forth to illustrate, but are not to be construed to limit, the present disclosure. It is obvious to a person skilled in the art that the examples described below may be modified without departing from the spirit of the disclosure.

### EXAMPLE 1. Generation of [Anti-TIGIT Monoclonal Antibody]-[Wild-Type IL-15] Fusion Protein (7A615, 2B715, & 3F815)

### 1.1. Expression and Purification of [Anti-TIGIT Monoclonal Antibody]-[Wild-Type IL-15] Fusion Protein

Fusion proteins in which an anti-TIGIT antibody and IL-15 were fused to each other, either directly or through a linker, was designed to have the structure shown in Table 3 below:

**TABLE 3**

| Fusion protein name | Structure | Linker |
|---|---|---|
| 7A615 | TIGIT mAb-(LC-(EAAAK)3-(IL-15)2 | Wild type IL-15 fused to LC C-terminus (LC variant) with rigid linker |
| (Fusion protein of TIGIT mAb 7A6 with wild type IL-15 at LC linking) | | |
| | TIGIT mAb-(LC-(GGGGS)3-(IL-15)2 | Wild type IL-15 fused to LC C-terminus (LC variant) with flexible linker |
| 7A615-Fc | TIGIT mAb-Fc-((EAAAK)3-(IL-15)2 | Wild type IL-15 fused to Fc C-terminus (Fc variant) with rigid linker |
| (Fusion protein of TIGIT mAb 7A6 with wild type IL-15 at FC linking) | | |
| | TIGIT mAb-Fc-((GGGGS)3-(IL-15)2 | Wild type IL-15 fused to Fc C-terminus (Fc variant) with flexible linker |
| | TIGIT mAb-Fc-(IL-15)2 | Wild type IL-15 fused to Fc C-terminus (Fc variant) without linker |
| 2B715 | TIGIT mAb-(LC-(EAAAK)3-IL-15)2 | Wild type IL-15 fused to LC C-terminus (LC variant) with rigid linker |
| (Fusion protein of TIGIT mAb 2B7 with wild type IL-15 at LC linking) | | |
| | TIGIT mAb-(LC-(GGGGS)3-IL-15)2 | Wild type IL-15 fused to LC C-terminus (LC variant) with flexible linker |
| 2B715-Fc | TIGIT mAb-Fc-((EAAAK)3-IL-15)2 | Wild type IL-15 fused to Fc C-terminus (Fc variant) with rigid linker |
| (Fusion protein of TIGIT mAb 2B7 with wild type IL-15 at FC linking) | | |
| | TIGIT mAb-Fc-((GGGGS)3-IL-15)2 | Wild type IL-15 fused to Fc C-terminus (Fc variant) with flexible linker |
| | TIGIT mAb-Fc-(IL-15)2 | Wild type IL-15 fused to Fc C-terminus (Fc variant) without linker |
| 3F815 | TIGIT mAb-(LC-(EAAAK)3-IL-15)2 | Wild type IL-15 fused to LC C-terminus (LC variant) with rigid linker |
| (Fusion protein of TIGIT mAb 3F8 with wild type IL-15 at LC linking) | | |
| | TIGIT mAb-(LC-(GGGGS)3-IL-15)2 | Wild type IL-15 fused to LC C-terminus (LC variant) with flexible linker |
| 3F815-Fc | TIGIT mAb-Fc-((EAAAK)3-IL-15)2 | Wild type IL-15 fused to Fc C-terminus (Fc variant) with rigid linker |
| (Fusion protein of TIGIT mAb 3F8 with wild type IL-15 at FC linking) | | |
| | TIGIT mAb-Fc-((GGGGS)3-IL-15)2 | Wild type IL-15 fused to Fc C-terminus (Fc variant) with flexible linker |
| | TIGIT mAb-Fc-(IL-15)2 | Wild type IL-15 fused to Fc C-terminus (Fc variant) without linker |

| | | |
|---|---|---|
| (In Table 3, Anti-TIGIT Monoclonal Antibody (TIGIT mAb): 7A6 (7A6_VH3/Vk5), 2B7, 3F8; IL-15: wild type IL-15; LC variant: IL-15 fused to the C-terminus of the light chain constant region (LC) of the antibody; Fc variants: IL-15 fused to the C-terminus of the heavy chain constant region (Fc; including the ΔK478 mutation) of the antibody.) | | |

For the production of the fusion proteins, the genes encoding the variable regions of the heavy and light chains of TIGIT mAb, as well as the genes encoding the constant regions of the heavy and light chains linked to [Linker]-[Wild-Type IL-15], were synthesized and cloned into the pcDNA3.1 (Invitrogen) vector. CHO-K1 cells (ATCC) were seeded onto plates and cultured to reach 70-90% confluence, after which 1 µg/µl of the DNA for each gene and Lipofectamine reagent (Thermo) were each diluted in Opti-MEM medium and mixed at a 1:1 ratio. After incubation for 5 minutes, the DNA-lipid complex was added to the CHO-K1 cells, and the cells were incubated at 37°C for transient expression. The culture supernatant was purified using Protein A and preparative SEC. The resulting protein was buffer-exchanged with PBS, quantified by OD280nm measurement, and analyzed by SDS-PAGE.

**TABLE 4**

| 7A615 (7A6 VH3/Vk5-IL-15) | | |
|---|---|---|
| Components | Amino acid sequence(N-C) or nucleic acid sequence(5'→3') | SEQ ID NO |
| TIGIT mAb | QVQLQESGPGLVKPSQTLSLTCTVTGYSITSDYAWNWI | 24 |
| heavy-chain variable region | RQPPGKGLEWMGYISYSGSARYNPSLKSRVTISRDTSK NQFSLKLSSVTAEDTATYYCARKGYPAYFAYWGQGTL VTVSS | |
| | CAGGTGCAGCTGCAGGAGAGCGGCCCCGGCCTGGTG AAGCCCAGCCAGACCCTGAGCCTGACCTGCACCGTG ACCGGCTACAGCATCACCAGCGACTACGCCTGGAAC TGGATCAGGCAGCCCCCCGGCAAGGGCCTGGAGTGG ATGGGCTACATCAGCTACAGCGGCAGCGCCAGGTAC AACCCCAGCCTGAAGAGCAGGGTGACCATCAGCAGG GACACCAGCAAGAACCAGTTCAGCCTGAAGCTGAGC AGCGTGACCGCCGAGGACACCGCCACCTACTACTGC GCCAGGAAGGGCTACCCCGCCTACTTCGCCTACTGG GGCCAGGGCACCCTGGTGACCGTGAGCAGC | 65 |
| TIGIT mAb heavy-chain constant region | ASTKGPSVFPLAPSSKSTSGGTAALGCLVKDYFPEPVTV SWNSGALTSGVHTFPAVLQSSGLYSLSSVVTVPSSSLGT QTYICNVNHKPSNTKVDKRVEPKSCDKTHTCPPCPAPE LLGGPSVFLFPPKPKDTLMISRTPEVTCVVVDVSHEDPE VKFNWYVDGVEVHNAKTKPREEQYNSTYRVVSVLTV LHQDWLNGKEYKCKVSNKALPAPIEKTISKAKGQPREP QVYTLPPSREEMTKNQVSLTCLVKGFYPSDIAVEWESN GQPENNYKTTPPVLDSDGSFFLYSKLTVDKSRWQQGN VFSCSVMHEALHNHYTQKSLSLSPGK | 41 |
| | GCCAGCACCAAGGGCCCCAGCGTGTTCCCCCTGGCC CCCAGCAGCAAGAGCACCAGCGGCGGCACCGCCGCC CTGGGCTGCCTGGTGAAGGACTACTTCCCCGAGCCC GTGACCGTGAGCTGGAACAGCGGCGCCCTGACCAGC GGCGTGCACACCTTCCCCGCCGTGCTGCAGAGCAGC GGCCTGTACAGCCTGAGCAGCGTGGTGACCGTGCCC AGCAGCAGCCTGGGCACCCAGACCTACATCTGCAAC GTGAACCACAAGCCCAGCAACACCAAGGTGGACAAG AGGGTGGAGCCCAAGAGCTGCGACAAGACCCACACC TGCCCCCCCTGCCCCGCCCCCGAGCTGCTGGGCGGCC CCAGCGTGTTCCTGTTCCCCCCCAAGCCCAAGGACAC CCTGATGATCAGCAGGACCCCCGAGGTGACCTGCGT GGTGGTGGACGTGAGCCACGAGGACCCCGAGGTGAA GTTCAACTGGTACGTGGACGGCGTGGAGGTGCACAA CGCCAAGACCAAGCCCAGGGAGGAGCAGTACAACA GCACCTACAGGGTGGTGAGCGTGCTGACCGTGCTGC ACCAGGACTGGCTGAACGGCAAGGAGTACAAGTGCA AGGTGAGCAACAAGGCCCTGCCCGCCCCCATCGAGA AGACCATCAGCAAGGCCAAGGGCCAGCCCAGGGAG CCCCAGGTGTACACCCTGCCCCCCAGCAGGGAGGAG ATGACCAAGAACCAGGTGAGCCTGACCTGCCTGGTG AAGGGCTTCTACCCCAGCGACATCGCCGTGGAGTGG GAGAGCAACGGCCAGCCCGAGAACAACTACAAGAC CACCCCCCCCGTGCTGGACAGCGACGGCAGCTTCTTC CTGTACAGCAAGCTGACCGTGGACAAGAGCAGGTGG CAGCAGGGCAACGTGTTCAGCTGCAGCGTGATGCAC GAGGCCCTGCACAACCACTACACCCAGAAGAGCCTG AGCCTGAGCCCCGGCAAG | 67 |
| TIGIT mAb heavy chain | QVQLQESGPGLVKPSQTLSLTCTVTGYSITSDYAWNWI RQPPGKGLEWMGYISYSGSARYNPSLKSRVTISRDTSK NQFSLKLSSVTAEDTATYYCARKGYPAYFAYWGQGTL VTVSSASTKGPSVFPLAPSSKSTSGGTAALGCLVKDYFP EPVTVSWNSGALTSGVHTFPAVLQSSGLYSLSSVVTVPS SSLGTQTYICNVNHKPSNTKVDKRVEPKSCDKTHTCPP CPAPELLGGPSVFLFPPKPKDTLMISRTPEVTCVVVDVS HEDPEVKFNWYVDGVEVHNAKTKPREEQYNSTYRVV SVLTVLHQDWLNGKEYKCKVSNKALPAPIEKTISKAKG QPREPQVYTLPPSREEMTKNQVSLTCLVKGFYPSDIAVE WESNGQPENNYKTTPPVLDSDGSFFLYSKLTVDKSRW QQGNVFSCSVMHEALHNHYTQKSLSLSPGK | 42 |
| TIGIT mAb light-chain variable region | DIQMTQSPSSLSASVGDRVSITCKASQDVSTAVAWYQQ KPGQAPRLLIYSASYRYTGVPDRFSGSGSGTDFTLTISSL QPEDFATYYCQHHYSTPYTFGQGTKLEIK | 34 |
| | GACATCCAGATGACCCAGAGCCCCAGCAGCCTGAGC GCCAGCGTGGGCGACAGGGTGAGCATCACCTGCAAG GCCAGCCAGGACGTGAGCACCGCCGTGGCCTGGTAC CAGCAGAAGCCCGGCCAGGCCCCCAGGCTGCTGATC TACAGCGCCAGCTACAGGTACACCGGCGTGCCCGAC AGGTTCAGCGGCAGCGGCAGCGGCACCGACTTCACC CTGACCATCAGCAGCCTGCAGCCCGAGGACTTCGCC ACCTACTACTGCCAGCACCACTACAGCACCCCCTACA CCTTCGGCCAGGGCACCAAGCTGGAGATCAAG | 66 |
| TIGIT mAb light-chain constant region | | 43 |
| | AGGACCGTGGCCGCCCCCAGCGTGTTCATCTTCCCCC CCAGCGACGAGCAGCTGAAGAGCGGCACCGCCAGC GTGGTGTGCCTGCTGAACAACTTCTACCCCAGGGAG GCCAAGGTGCAGTGGAAGGTGGACAACGCCCTGCAG AGCGGCAACAGCCAGGAGAGCGTGACCGAGCAGGA CAGCAAGGACAGCACCTACAGCCTGAGCAGCACCCT GACCCTGAGCAAGGCCGACTACGAGAAGCACAAGGT GTACGCCTGCGAGGTGACCCACCAGGGCCTGAGCAG CCCCGTGACCAAGAGCTTCAACAGGGGCGAGTGC | 68 |
| TIGIT mAb light chain | DIQMTQSPSSLSASVGDRVSITCKASQDVSTAVAWYQQ KPGQAPRLLIYSASYRYTGVPDRFSGSGSGTDFTLTISSL QPEDFATYYCQHHYSTPYTFGQGTKLEIKRTVAAPSVFI FPPSDEQLKSGTASVVCLLNNFYPREAKVQWKVDNAL QSGNSQESVTEQDSKDSTYSLSSTLTLSKADYEKHKVY ACEVTHQGLSSPVTKSFNRGEC | 44 |
| IL-15 polypeptide | NWVNVISDLKKIEDLIQSMHIDATLYTESDVHPSCKVTA MKCFLLELQVISLESGDASIHDTVENLIILANNSLSSNGN VTESGCKECEELEEKNIKEFLQSFVHIVQMFINTS | 38 |
| | AACTGGGTGAACGTGATCAGCGACCTGAAGAAGATC GAGGACCTGATCCAGAGCATGCACATCGACGCCACC CTGTACACCGAGAGCGACGTGCACCCCAGCTGCAAG GTGACCGCCATGAAGTGCTTCCTGCTGGAGCTGCAG GTGATCAGCCTGGAGAGCGGCGACGCCAGCATCCAC GACACCGTGGAGAACCTGATCATCCTGGCCAACAAC AGCCTGAGCAGCAACGGCAACGTGACCGAGAGCGGC TGCAAGGAGTGCGAGGAGCTGGAGGAGAAGAACAT CAAGGAGTTCCTGCAGAGCTTCGTGCACATCGTGCA GATGTTCATCAACACCAGC | 69 |
| Flexible linker (V-1) | GGGGSGGGGSGGGGS | 39 |
| | GGCGGCGGCGGCAGCGGCGGCGGCGGCAGCGGCGG CGGCGGCAGC | 70 |
| Rigid linker (V-2) | EAAAKEAAAKEAAAK | 40 |
| | GAGGCCGCCGCCAAGGAGGCCGCCGCCAAGGAGGC CGCCGCCAAG | 71 |
| **7A615 TL-1 (IL-15 (2 copies) connected to each C terminus of the light chain (2 copies) of anti-TIGIT antibody through linker V-1)** | | |
| Heavy-chain strand (TIGIT mAb heavy chain(IgG1)) | QVQLQESGPGLVKPSQTLSLTCTVTGYSITSDYAWNWI RQPPGKGLEWMGYISYSGSARYNPSLKSRVTISRDTSK NQFSLKLSSVTAEDTATYYCARKGYPAYFAYWGQGTL VTVSSASTKGPSVFPLAPSSKSTSGGTAALGCLVKDYFP EPVTVSWNSGALTSGVHTFPAVLQSSGLYSLSSVVTVPS SSLGTQTYICNVNHKPSNTKVDKRVEPKSCDKTHTCPP CPAPELLGGPSVFLFPPKPKDTLMISRTPEVTCVVVDVS HEDPEVKFNWYVDGVEVHNAKTKPREEQYNSTYRVV SVLTVLHQDWLNGKEYKCKVSNKALPAPIEKTISKAKG QPREPQVYTLPPSREEMTKNQVSLTCLVKGFYPSDIAVE WESNGQPENNYKTTPPVLDSDGSFFLYSKLTVDKSRW QQGNVFSCSVMHEALHNHYTQKSLSLSPGK | 42 |
| Light-chain strand ([TIGIT mAb light chain(kappk)]-[linker(V-1)]-[IL-15]) | DIQMTQSPSSLSASVGDRVSITCKASQDVSTAVAWYQQ KPGQAPRLLIYSASYRYTGVPDRFSGSGSGTDFTLTISSL QPEDFATYYCQHHYSTPYTFGQGTKLEIKRTVAAPSVFI FPPSDEQLKSGTASVVCLLNNFYPREAKVQWKVDNAL QSGNSQESVTEQDSKDSTYSLSSTLTLSKADYEKHKVY ACEVTHQGLSSPVTKSFNRGECGGGGSGGGGSGGGGS NWVNVISDLKKIEDLIQSMHIDATLYTESDVHPSCKVTA MKCFLLELQVISLESGDASIHDTVENLIILANNSLSSNGN VTESGCKECEELEEKNIKEFLQSFVHIVQMFINTS | 45 |
| **7A615 TL-2 (IL-15 (2 copies) connected to each C terminus of the light chain (2 copies) of anti-TIGIT antibody through linker V-2)** | | |
| Heavy-chain strand (TIGIT mAb heavy chain(IgG1)) | QVQLQESGPGLVKPSQTLSLTCTVTGYSITSDYAWNWI RQPPGKGLEWMGYISYSGSARYNPSLKSRVTISRDTSK NQFSLKLSSVTAEDTATYYCARKGYPAYFAYWGQGTL VTVSSASTKGPSVFPLAPSSKSTSGGTAALGCLVKDYFP EPVTVSWNSGALTSGVHTFPAVLQSSGLYSLSSVVTVPS SSLGTQTYICNVNHKPSNTKVDKRVEPKSCDKTHTCPP CPAPELLGGPSVFLFPPKPKDTLMISRTPEVTCVVVDVS | 42 |
| Light-chain strand ([TIGIT mAb light chain(kappk)]-[linker(V-2)]-[IL-15]) | DIQMTQSPSSLSASVGDRVSITCKASQDVSTAVAWYQQ KPGQAPRLLIYSASYRYTGVPDRFSGSGSGTDFTLTISSL QPEDFATYYCQHHYSTPYTFGQGTKLEIKRTVAAPSVFI FPPSDEQLKSGTASVVCLLNNFYPREAKVQWKVDNAL QSGNSQESVTEQDSKDSTYSLSSTLTLSKADYEKHKVY ACEVTHQGLSSPVTKSFNRGECEAAAKEAAAKEAAAK NWVNVISDLKKIEDLIQSMHIDATLYTESDVHPSCKVTA MKCFLLELQVISLESGDASIHDTVENLIILANNSLSSNGN VTESGCKECEELEEKNIKEFLQSFVHIVQMFINTS | 46 |
| **7A615 TL-3 (IL-15 (2 copies) connected to each C terminus of the heavy chain (2 copies) of anti-TIGIT antibody through linker V-1)** | | |
| Heavy-chain strand ([TIGIT mAb heavy chain(IgG1)]-[linker(V-1)]-[IL-15]) | QVQLQESGPGLVKPSQTLSLTCTVTGYSITSDYAWNWI RQPPGKGLEWMGYISYSGSARYNPSLKSRVTISRDTSK NQFSLKLSSVTAEDTATYYCARKGYPAYFAYWGQGTL VTVSSASTKGPSVFPLAPSSKSTSGGTAALGCLVKDYFP EPVTVSWNSGALTSGVHTFPAVLQSSGLYSLSSVVTVPS SSLGTQTYICNVNHKPSNTKVDKRVEPKSCDKTHTCPP CPAPELLGGPSVFLFPPKPKDTLMISRTPEVTCVVVDVS HEDPEVKFNWYVDGVEVHNAKTKPREEQYNSTYRVV SVLTVLHQDWLNGKEYKCKVSNKALPAPIEKTISKAKG QPREPQVYTLPPSREEMTKNQVSLTCLVKGFYPSDIAVE WESNGQPENNYKTTPPVLDSDGSFFLYSKLTVDKSRW QQGNVFSCSVMHEALHNHYTQKSLSLSPGKGGGGSGG GGSGGGGSNWVNVISDLKKIEDLIQSMHIDATLYTESD VHPSCKVTAMKCFLLELQVISLESGDASIHDTVENLIILA NNSLSSNGNVTESGCKECEELEEKNIKEFLQSFVHIVQM FINTS | 47 |
| Light-chain strand (TIGIT mAb light chain(kappk)) | DIQMTQSPSSLSASVGDRVSITCKASQDVSTAVAWYQQ KPGQAPRLLIYSASYRYTGVPDRFSGSGSGTDFTLTISSL QPEDFATYYCQHHYSTPYTFGQGTKLEIKRTVAAPSVFI FPPSDEQLKSGTASVVCLLNNFYPREAKVQWKVDNAL QSGNSQESVTEQDSKDSTYSLSSTLTLSKADYEKHKVY ACEVTHQGLSSPVTKSFNRGEC | 44 |
| **7A615 TL-4 (IL-15 (2 copies) connected to each C terminus of the heavy chain (2 copies)** of **anti-TIGIT antibody through linker V-2)** | | |
| Heavy-chain strand ([TIGIT mAb heavy chain(IgG1)]-[linker(V-2)]-[IL-15]) | QVQLQESGPGLVKPSQTLSLTCTVTGYSITSDYAWNWI RQPPGKGLEWMGYISYSGSARYNPSLKSRVTISRDTSK NQFSLKLSSVTAEDTATYYCARKGYPAYFAYWGQGTL VTVSSASTKGPSVFPLAPSSKSTSGGTAALGCLVKDYFP EPVTVSWNSGALTSGVHTFPAVLQSSGLYSLSSVVTVPS SSLGTQTYICNVNHKPSNTKVDKRVEPKSCDKTHTCPP CPAPELLGGPSVFLFPPKPKDTLMISRTPEVTCVVVDVS HEDPEVKFNWYVDGVEVHNAKTKPREEQYNSTYRVV SVLTVLHQDWLNGKEYKCKVSNKALPAPIEKTISKAKG QPREPQVYTLPPSREEMTKNQVSLTCLVKGFYPSDIAVE WESNGQPENNYKTTPPVLDSDGSFFLYSKLTVDKSRW QQGNVFSCSVMHEALHNHYTQKSLSLSPGKEAAAKEA AAKEAAAKNWVNVISDLKKIEDLIQSMHIDATLYTESD VHPSCKVTAMKCFLLELQVISLESGDASIHDTVENLIILA NNSLSSNGNVTESGCKECEELEEKNIKEFLQSFVHIVQM FINTS | 48 |
| Light-chain strand (TIGIT mAb light chain(kappk)) | DIQMTQSPSSLSASVGDRVSITCKASQDVSTAVAWYQQ KPGQAPRLLIYSASYRYTGVPDRFSGSGSGTDFTLTISSL QPEDFATYYCQHHYSTPYTFGQGTKLEIKRTVAAPSVFI FPPSDEQLKSGTASVVCLLNNFYPREAKVQWKVDNAL QSGNSQESVTEQDSKDSTYSLSSTLTLSKADYEKHKVY ACEVTHQGLSSPVTKSFNRGEC | 44 |
| **7A615 TL-5** (**IL-15 (2 copies) connected to each C terminus of the heavy chain (2 copies) of anti-TIGIT antibody without linker** | | |
| Heavy-chain strand ([TIGIT mAb heavy chain(IgG1)]-[IL-15]) | QVQLQESGPGLVKPSQTLSLTCTVTGYSITSDYAWNWI RQPPGKGLEWMGYISYSGSARYNPSLKSRVTISRDTSK NQFSLKLSSVTAEDTATYYCARKGYPAYFAYWGQGTL VTVSSASTKGPSVFPLAPSSKSTSGGTAALGCLVKDYFP EPVTVSWNSGALTSGVHTFPAVLQSSGLYSLSSVVTVPS SSLGTQTYICNVNHKPSNTKVDKRVEPKSCDKTHTCPP CPAPELLGGPSVFLFPPKPKDTLMISRTPEVTCVVVDVS HEDPEVKFNWYVDGVEVHNAKTKPREEQYNSTYRVV SVLTVLHQDWLNGKEYKCKVSNKALPAPIEKTISKAKG QPREPQVYTLPPSREEMTKNQVSLTCLVKGFYPSDIAVE WESNGQPENNYKTTPPVLDSDGSFFLYSKLTVDKSRW QQGNVFSCSVMHEALHNHYTQKSLSLSPGKNWVNVIS DLKKIEDLIQSMHIDATLYTESDVHPSCKVTAMKCFLLE LQVISLESGDASIHDTVENLIILANNSLSSNGNVTESGCK ECEELEEKNIKEFLQSFVHIVQMFINTS | 49 |
| Light-chain strand (TIGIT mAb light chain(kappk)) | DIQMTQSPSSLSASVGDRVSITCKASQDVSTAVAWYQQ KPGQAPRLLIYSASYRYTGVPDRFSGSGSGTDFTLTISSL QPEDFATYYCQHHYSTPYTFGQGTKLEIKRTVAAPSVFI FPPSDEQLKSGTASVVCLLNNFYPREAKVQWKVDNAL QSGNSQESVTEQDSKDSTYSLSSTLTLSKADYEKHKVY ACEVTHQGLSSPVTKSFNRGEC | 44 |

**TABLE 5**

| 2B715 | | |
|---|---|---|
| Components | Amino acid sequence (N→C) or Nucleic acid sequence (5'→3') | SEQ ID NO: |
| TIGIT mAb heavy-chain variable region | EVQLQQSGPELVKPGASVKISCKTSGSTFTEYTMHWV KQSHGKSLEWIGGLNPNNGGTSYNQRFKDRATLTVDK SSSTAYMELRSLTSEDSAVYYCTRGTYYDYSFAYWGQ GTLVTVSA | 27 |
| | GAGGTGCAGCTGCAGCAGAGCGGCCCCGAGCTGGT GAAGCCCGGCGCCAGCGTGAAGATCAGCTGCAAGA CCAGCGGCAGCACCTTCACCGAGTACACCATGCACT GGGTGAAGCAGAGCCACGGCAAGAGCCTGGAGTGG ATCGGCGGCCTGAACCCCAACAACGGCGGCACCAG CTACAACCAGAGGTTCAAGGACAGGGCCACCCTGAC CGTGGACAAGAGCAGCAGCACCGCCTACATGGAGC TGAGGAGCCTGACCAGCGAGGACAGCGCCGTGTACT ACTGCACCAGGGGCACCTACTACGACTACAGCTTCG CCTACTGGGGCCAGGGCACCCTGGTGACCGTGAGCG CC | 72 |
| TIGIT mAb heavy-chain constant region | ASTKGPSVFPLAPSSKSTSGGTAALGCLVKDYFPEPVT VSWNSGALTSGVHTFPAVLQSSGLYSLSSVVTVPSSSL GTQTYICNVNHKPSNTKVDKRVEPKSCDKTHTCPPCPA PELLGGPSVFLFPPKPKDTLMISRTPEVTCVVVDVSHED PEVKFNWYVDGVEVHNAKTKPREEQYNSTYRVVSVL TVLHQDWLNGKEYKCKVSNKALPAPIEKTISKAKGQP REPQVYTLPPSREEMTKNQVSLTCLVKGFYPSDIAVEW ESNGQPENNYKTTPPVLDSDGSFFLYSKLTVDKSRWQ QGNVFSCSVMHEALHNHYTQKSLSLSPGK | 41 |
| TIGIT mAb heavy chain | EVQLQQSGPELVKPGASVKISCKTSGSTFTEYTMHWV KQSHGKSLEWIGGLNPNNGGTSYNQRFKDRATLTVDK SSSTAYMELRSLTSEDSAVYYCTRGTYYDYSFAYWGQ GTLVTVSAASTKGPSVFPLAPSSKSTSGGTAALGCLVK DYFPEPVTVSWNSGALTSGVHTFPAVLQSSGLYSLSSV VTVPSSSLGTQTYICNVNHKPSNTKVDKRVEPKSCDKT HTCPPCPAPELLGGPSVFLFPPKPKDTLMISRTPEVTCV VVDVSHEDPEVKFNWYVDGVEVHNAKTKPREEQYNS TYRVVSVLTVLHQDWLNGKEYKCKVSNKALPAPIEKT ISKAKGQPREPQVYTLPPSREEMTKNQVSLTCLVKGFY PSDIAVEWESNGQPENNYKTTPPVLDSDGSFFLYSKLT VDKSRWQQGNVFSCSVMHEALHNHYTQKSLSLSPGK | 50 |
| TIGIT mAb light-chain variable region | DIVMTQSHKFMSTSVGDRVSITCKASHYVSTAVAWYQ QKPGQSPKLLIYSPSYRYTGVPDRFTGSGSGTDFTFTISS VQAEDLAVYYCHQHYSTPWTFGGGTKLEIK | 35 |
| | GACATCGTGATGACCCAGAGCCACAAGTTCATGAGC ACCAGCGTGGGCGACAGGGTGAGCATCACCTGCAA GGCCAGCCACTACGTGAGCACCGCCGTGGCCTGGTA CCAGCAGAAGCCCGGCCAGAGCCCCAAGCTGCTGAT CTACAGCCCCAGCTACAGGTACACCGGCGTGCCCGA CAGGTTCACCGGCAGCGGCAGCGGCACCGACTTCAC CTTCACCATCAGCAGCGTGCAGGCCGAGGACCTGGC CGTGTACTACTGCCACCAGCACTACAGCACCCCCTG GACCTTCGGCGGCGGCACCAAGCTGGAGATCAAG | 73 |
| TIGIT mAb light-chain constant region | | 43 |
| | | |
| TIGIT mAb light chain | DIVMTQSHKFMSTSVGDRVSITCKASHYVSTAVAWYQ QKPGQSPKLLIYSPSYRYTGVPDRFTGSGSGTDFTFTISS VQAEDLAVYYCHQHYSTPWTFGGGTKLEIKRTVAAPS VFIFPPSDEQLKSGTASVVCLLNNFYPREAKVQWKVD NALQSGNSQESVTEQDSKDSTYSLSSTLTLSKADYEKH KVYACEVTHQGLSSPVTKSFNRGEC | 51 |
| IL-15 polypeptide | NWVNVISDLKKIEDLIQSMHIDATLYTESDVHPSCKVT AMKCFLLELQVISLESGDASIHDTVENLIILANNSLSSNG NVTESGCKECEELEEKNIKEFLQSFVHIVQMFINTS | 38 |
| Flexible linker (V-1) | GGGGSGGGGSGGGGS | 39 |
| Rigid linker (V-2) | EAAAKEAAAKEAAAK | 40 |

| **2B715 TL-1** | | |
|---|---|---|
| **(IL-15 (2 copies) connected to each C terminus of the light chain (2 copies) of anti-TIGIT antibody through linker V-1)** | | |
| Heavy-chain strand (TIGIT mAb heavy chain(IgG1)) | EVQLQQSGPELVKPGASVKISCKTSGSTFTEYTMHWV KQSHGKSLEWIGGLNPNNGGTSYNQRFKDRATLTVDK SSSTAYMELRSLTSEDSAVYYCTRGTYYDYSFAYWGQ GTLVTVSAASTKGPSVFPLAPSSKSTSGGTAALGCLVK DYFPEPVTVSWNSGALTSGVHTFPAVLQSSGLYSLSSV VTVPSSSLGTQTYICNVNHKPSNTKVDKRVEPKSCDKT HTCPPCPAPELLGGPSVFLFPPKPKDTLMISRTPEVTCV VVDVSHEDPEVKFNWYVDGVEVHNAKTKPREEQYNS TYRVVSVLTVLHQDWLNGKEYKCKVSNKALPAPIEKT ISKAKGQPREPQVYTLPPSREEMTKNQVSLTCLVKGFY PSDIAVEWESNGQPENNYKTTPPVLDSDGSFFLYSKLT VDKSRWQQGNVFSCSVMHEALHNHYTQKSLSLSPGK | 50 |
| Light-chain strand ([TIGIT mAb light chain(kappk)]-[linker(V-1)]-[IL-15]) | DIVMTQSHKFMSTSVGDRVSITCKASHYVSTAVAWYQ QKPGQSPKLLIYSPSYRYTGVPDRFTGSGSGTDFTFTISS VQAEDLAVYYCHQHYSTPWTFGGGTKLEIKRTVAAPS VFIFPPSDEQLKSGTASVVCLLNNFYPREAKVQWKVD NALQSGNSQESVTEQDSKDSTYSLSSTLTLSKADYEKH KVYACEVTHQGLSSPVTKSFNRGECGGGGSGGGGSGG GGSNWVNVISDLKKIEDLIQSMHIDATLYTESDVHPSC KVTAMKCFLLELQVISLESGDASIHDTVENLIILANNSL | 52 |

| **2B715 TL-2** | | |
|---|---|---|
| **(IL-15 (2 copies) connected to each C terminus of the light chain (2 copies) of anti-TIGIT antibody through linker V-2)** | | |
| Heavy-chain strand (TIGIT mAb heavy chain(IgG1)) | EVQLQQSGPELVKPGASVKISCKTSGSTFTEYTMHWV KQSHGKSLEWIGGLNPNNGGTSYNQRFKDRATLTVDK SSSTAYMELRSLTSEDSAVYYCTRGTYYDYSFAYWGQ GTLVTVSAASTKGPSVFPLAPSSKSTSGGTAALGCLVK DYFPEPVTVSWNSGALTSGVHTFPAVLQSSGLYSLSSV VTVPSSSLGTQTYICNVNHKPSNTKVDKRVEPKSCDKT HTCPPCPAPELLGGPSVFLFPPKPKDTLMISRTPEVTCV VVDVSHEDPEVKFNWYVDGVEVHNAKTKPREEQYNS TYRVVSVLTVLHQDWLNGKEYKCKVSNKALPAPIEKT ISKAKGQPREPQVYTLPPSREEMTKNQVSLTCLVKGFY PSDIAVEWESNGQPENNYKTTPPVLDSDGSFFLYSKLT VDKSRWQQGNVFSCSVMHEALHNHYTQKSLSLSPGK | 50 |
| Light-chain strand ([TIGIT mAb light chain(kappk)]-[linker (V-2)]-[IL-15]) | DIVMTQSHKFMSTSVGDRVSITCKASHYVSTAVAWYQ QKPGQSPKLLIYSPSYRYTGVPDRFTGSGSGTDFTFTISS VQAEDLAVYYCHQHYSTPWTFGGGTKLEIKRTVAAPS VFIFPPSDEQLKSGTASVVCLLNNFYPREAKVQWKVD NALQSGNSQESVTEQDSKDSTYSLSSTLTLSKADYEKH KVYACEVTHQGLSSPVTKSFNRGECEAAAKEAAAKEA AAKNWVNVISDLKKIEDLIQSMHIDATLYTESDVHPSC KVTAMKCFLLELQVISLESGDASIHDTVENLIILANNSL SSNGNVTESGCKECEELEEKNIKEFLQSFVHIVQMFINT S | 53 |

| **2B715 TL-3** | | |
|---|---|---|
| **(IL-15 (2 copies) connected to each C terminus of the heavy chain (2 copies) of anti-TIGIT antibody through linker V-1)** | | |
| Heavy-chain strand ([TIGIT mAb heavy chain(IgG1)]- | EVQLQQSGPELVKPGASVKISCKTSGSTFTEYTMHWV KQSHGKSLEWIGGLNPNNGGTSYNQRFKDRATLTVDK SSSTAYMELRSLTSEDSAVYYCTRGTYYDYSFAYWGQ GTLVTVSAASTKGPSVFPLAPSSKSTSGGTAALGCLVK | 54 |
| [linker(V-1)]-[IL-15]) | DYFPEPVTVSWNSGALTSGVHTFPAVLQSSGLYSLSSV VTVPSSSLGTQTYICNVNHKPSNTKVDKRVEPKSCDKT HTCPPCPAPELLGGPSVFLFPPKPKDTLMISRTPEVTCV VVDVSHEDPEVKFNWYVDGVEVHNAKTKPREEQYNS TYRVVSVLTVLHQDWLNGKEYKCKVSNKALPAPIEKT ISKAKGQPREPQVYTLPPSREEMTKNQVSLTCLVKGFY PSDIAVEWESNGQPENNYKTTPPVLDSDGSFFLYSKLT VDKSRWQQGNVFSCSVMHEALHNHYTQKSLSLSPGK GGGGSGGGGSGGGGSNWVNVISDLKKIEDLIQSMHID ATLYTESDVHPSCKVTAMKCFLLELQVISLESGDASIH DTVENLIILANNSLSSNGNVTESGCKECEELEEKNIKEF LQSFVHIVQMFINTS | |
| Light-chain strand (TIGIT mAb light chain(kappk)) | DIVMTQSHKFMSTSVGDRVSITCKASHYVSTAVAWYQ QKPGQSPKLLIYSPSYRYTGVPDRFTGSGSGTDFTFTISS VQAEDLAVYYCHQHYSTPWTFGGGTKLEIKRTVAAPS VFIFPPSDEQLKSGTASVVCLLNNFYPREAKVQWKVD NALQSGNSQESVTEQDSKDSTYSLSSTLTLSKADYEKH KVYACEVTHQGLSSPVTKSFNRGEC | 51 |

| **2B715 TL-4** | | |
|---|---|---|
| **(IL-15 (2 copies) connected to each C terminus of the heavy chain (2 copies) of anti-TIGIT antibody through linker V-2)** | | |
| Heavy-chain strand ([TIGIT mAb heavy chain(IgG1)]-[linker(V-2)]-[IL-15]) | EVQLQQSGPELVKPGASVKISCKTSGSTFTEYTMHWV KQSHGKSLEWIGGLNPNNGGTSYNQRFKDRATLTVDK SSSTAYMELRSLTSEDSAVYYCTRGTYYDYSFAYWGQ GTLVTVSAASTKGPSVFPLAPSSKSTSGGTAALGCLVK DYFPEPVTVSWNSGALTSGVHTFPAVLQSSGLYSLSSV VTVPSSSLGTQTYICNVNHKPSNTKVDKRVEPKSCDKT HTCPPCPAPELLGGPSVFLFPPKPKDTLMISRTPEVTCV VVDVSHEDPEVKFNWYVDGVEVHNAKTKPREEQYNS TYRVVSVLTVLHQDWLNGKEYKCKVSNKALPAPIEKT ISKAKGQPREPQVYTLPPSREEMTKNQVSLTCLVKGFY PSDIAVEWESNGQPENNYKTTPPVLDSDGSFFLYSKLT VDKSRWQQGNVFSCSVMHEALHNHYTQKSLSLSPGK EAAAKEAAAKEAAAKNWVNVISDLKKIEDLIQSMHID ATLYTESDVHPSCKVTAMKCFLLELQVISLESGDASIH DTVENLIILANNSLSSNGNVTESGCKECEELEEKNIKEF LQSFVHIVQMFINTS | 55 |
| Light-chain strand (TIGIT mAb light chain(kappk)) | DIVMTQSHKFMSTSVGDRVSITCKASHYVSTAVAWYQ QKPGQSPKLLIYSPSYRYTGVPDRFTGSGSGTDFTFTISS VQAEDLAVYYCHQHYSTPWTFGGGTKLEIKRTVAAPS VFIFPPSDEQLKSGTASVVCLLNNFYPREAKVQWKVD NALQSGNSQESVTEQDSKDSTYSLSSTLTLSKADYEKH KVYACEVTHQGLSSPVTKSFNRGEC | 51 |

| **2B715 TL-5** | | |
|---|---|---|
| **(IL-15 (2 copies) connected to each C terminus of the heavy chain (2 copies) of anti-TIGIT antibody without linker)** | | |
| Heay-chain strand ([TIGIT mAb heavy chain (IgG1)]-[IL-15]) | EVQLQQSGPELVKPGASVKISCKTSGSTFTEYTMHWV KQSHGKSLEWIGGLNPNNGGTSYNQRFKDRATLTVDK SSSTAYMELRSLTSEDSAVYYCTRGTYYDYSFAYWGQ GTLVTVSAASTKGPSVFPLAPSSKSTSGGTAALGCLVK DYFPEPVTVSWNSGALTSGVHTFPAVLQSSGLYSLSSV VTVPSSSLGTQTYICNVNHKPSNTKVDKRVEPKSCDKT HTCPPCPAPELLGGPSVFLFPPKPKDTLMISRTPEVTCV VVDVSHEDPEVKFNWYVDGVEVHNAKTKPREEQYNS TYRVVSVLTVLHQDWLNGKEYKCKVSNKALPAPIEKT ISKAKGQPREPQVYTLPPSREEMTKNQVSLTCLVKGFY PSDIAVEWESNGQPENNYKTTPPVLDSDGSFFLYSKLT VDKSRWQQGNVFSCSVMHEALHNHYTQKSLSLSPGK NWVNVISDLKKIEDLIQSMHIDATLYTESDVHPSCKVT AMKCFLLELQVISLESGDASIHDTVENLIILANNSLSSNG NVTESGCKECEELEEKNIKEFLQSFVHIVQMFINTS | 56 |
| Light-chain strand (TIGIT mAb light chain (kappk)) | DIVMTQSHKFMSTSVGDRVSITCKASHYVSTAVAWYQ QKPGQSPKLLIYSPSYRYTGVPDRFTGSGSGTDFTFTISS VQAEDLAVYYCHQHYSTPWTFGGGTKLEIKRTVAAPS VFIFPPSDEQLKSGTASVVCLLNNFYPREAKVQWKVD NALQSGNSQESVTEQDSKDSTYSLSSTLTLSKADYEKH KVYACEVTHQGLSSPVTKSFNRGEC | 51 |

**TABLE 6**

| 3F815 | | |
|---|---|---|
| Components | Amino acid sequence (N→C) or Nucleic acid sequence (5'→3') | SEQ ID NO: |
| TIGIT mAb heavy-chain variable region | EVQLQQSGPELVKPGASMKISCKASGYSFTDYIMNWVKQS HGKNLEWIGLSIPYNGGTSYNQKFEGKATLTVDKSSSTAYM ELLSLTSEDSAVYYCARGIKGYFAMDYWGQGTSVTVSS | 28 |
| | GAGGTGCAGCTGCAGCAGAGCGGCCCCGAGCTGGTGAA GCCCGGCGCCAGCATGAAGATCAGCTGCAAGGCCAGCG GCTACAGCTTCACCGACTACATCATGAACTGGGTGAAGC AGAGCCACGGCAAGAACCTGGAGTGGATCGGCCTGAGC ATCCCCTACAACGGCGGCACCAGCTACAACCAGAAGTTC GAGGGCAAGGCCACCCTGACCGTGGACAAGAGCAGCAG CACCGCCTACATGGAGCTGCTGAGCCTGACCAGCGAGGA CAGCGCCGTGTACTACTGCGCCAGGGGCATCAAGGGCTA CTTCGCCATGGACTACTGGGGCCAGGGCACCAGCGTGAC CGTGAGCAGC | 74 |
| TIGIT mAb heavy-chain constant region | ASTKGPSVFPLAPSSKSTSGGTAALGCLVKDYFPEPVTVSW NSGALTSGVHTFPAVLQSSGLYSLSSVVTVPSSSLGTQTYIC NVNHKPSNTKVDKRVEPKSCDKTHTCPPCPAPELLGGPSVF LFPPKPKDTLMISRTPEVTCVVVDVSHEDPEVKFNWYVDG VEVHNAKTKPREEQYNSTYRVVSVLTVLHQDWLNGKEYK CKVSNKALPAPIEKTISKAKGQPREPQVYTLPPSREEMTKNQ VSLTCLVKGFYPSDIAVEWESNGQPENNYKTTPPVLDSDGS FFLYSKLTVDKSRWQQGNVFSCSVMHEALHNHYTQKSLSL SPGK | 41 |
| TIGIT mAb heavy chain | EVQLQQSGPELVKPGASMKISCKASGYSFTDYIMNWVKQS HGKNLEWIGLSIPYNGGTSYNQKFEGKATLTVDKSSSTAYM ELLSLTSEDSAVYYCARGIKGYFAMDYWGQGTSVTVSSAS TKGPSVFPLAPSSKSTSGGTAALGCLVKDYFPEPVTVSWNS GALTSGVHTFPAVLQSSGLYSLSSVVTVPSSSLGTQTYICNV NHKPSNTKVDKRVEPKSCDKTHTCPPCPAPELLGGPSVFLFP PKPKDTLMISRTPEVTCVVVDVSHEDPEVKFNWYVDGVEV HNAKTKPREEQYNSTYRVVSVLTVLHQDWLNGKEYKCKV SNKALPAPIEKTISKAKGQPREPQVYTLPPSREEMTKNQVSL TCLVKGFYPSDIAVEWESNGQPENNYKTTPPVLDSDGSFFL YSKLTVDKSRWQQGNVFSCSVMHEALHNHYTQKSLSLSPG K | 57 |
| TIGIT mAb | DVVVTQTPLSLPVSFGDQVSISCRSSQSLVNSFGKTYLSWFL | 36 |
| light-chain variable region | HKPGQSPQLIMYGVSNRFSGVPDRFSGSGSGTDFTLKISTIKP EDLGMYYCLQGTHQPWTFGGGTKLEIK | |
| | GACGTGGTGGTGACCCAGACCCCCCTGAGCCTGCCCGTG AGCTTCGGCGACCAGGTGAGCATCAGCTGCAGGAGCAGC CAGAGCCTGGTGAACAGCTTCGGCAAGACCTACCTGAGC TGGTTCCTGCACAAGCCCGGCCAGAGCCCCCAGCTGATC ATGTACGGCGTGAGCAACAGGTTCAGCGGCGTGCCCGAC AGGTTCAGCGGCAGCGGCAGCGGCACCGACTTCACCCTG AAGATCAGCACCATCAAGCCCGAGGACCTGGGCATGTAC TACTGCCTGCAGGGCACCCACCAGCCCTGGACCTTCGGC GGCGGCACCAAGCTGGAGATCAAG | 75 |
| TIGIT mAb light-chain constant region | | 43 |
| TIGIT mAb light chain | DVVVTQTPLSLPVSFGDQVSISCRSSQSLVNSFGKTYLSWFL HKPGQSPQLIMYGVSNRFSGVPDRFSGSGSGTDFTLKISTIKP EDLGMYYCLQGTHQPWTFGGGTKLEIKRTVAAPSVFIFPPS DEQLKSGTASVVCLLNNFYPREAKVQWKVDNALQSGNSQ ESVTEQDSKDSTYSLSSTLTLSKADYEKHKVYACEVTHQGL SSPVTKSFNRGEC | 58 |
| IL-15 polypeptide | NWVNVISDLKKIEDLIQSMHIDATLYTESDVHPSCKVTAMK CFLLELQVISLESGDASIHDTVENLIILANNSLSSNGNVTESG CKECEELEEKNIKEFLQSFVHIVQMFINTS | 38 |
| Flexible linker (V-1) | GGGGSGGGGSGGGGS | 39 |
| Rigid linker (V-2) | EAAAKEAAAKEAAAK | 40 |

| **3F815 TL-1 (IL-15 (2 copies) connected to each C terminus of the light chain (2 copies) of anti-TIGIT antibody throughl inker V-1)** | | |
|---|---|---|
| Heavy-chain strand (TIGIT mAb heavy chain (IgG1)) | EVQLQQSGPELVKPGASMKISCKASGYSFTDYIMNWVK QSHGKNLEWIGLSIPYNGGTSYNQKFEGKATLTVDKSSS TAYMELLSLTSEDSAVYYCARGIKGYFAMDYWGQGTSV TVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKRVEPKSCDKTHTCPPCPAP ELLGGPSVFLFPPKPKDTLMISRTPEVTCVVVDVSHEDPE VKFNWYVDGVEVHNAKTKPREEQYNSTYRVVSVLTVL HQDWLNGKEYKCKVSNKALPAPIEKTISKAKGQPREPQV YTLPPSREEMTKNQVSLTCLVKGFYPSDIAVEWESNGQP ENNYKTTPPVLDSDGSFFLYSKLTVDKSRWQQGNVFSCS VMHEALHNHYTQKSLSLSPGK | 57 |
| Light-chain strand ([TIGIT mAb light chain(kappk)]-[linker(V-1)]-[IL-15]) | DVVVTQTPLSLPVSFGDQVSISCRSSQSLVNSFGKTYLSW FLHKPGQSPQLIMYGVSNRFSGVPDRFSGSGSGTDFTLKI STIKPEDLGMYYCLQGTHQPWTFGGGTKLEIKRTVAAPS VFIFPPSDEQLKSGTASVVCLLNNFYPREAKVQWKVDNA LQSGNSQESVTEQDSKDSTYSLSSTLTLSKADYEKHKVY ACEVTHQGLSSPVTKSFNRGECGGGGSGGGGSGGGGSN WVNVISDLKKIEDLIQSMHIDATLYTESDVHPSCKVTAM KCFLLELQVISLESGDASIHDTVENLIILANNSLSSNGNVT ESGCKECEELEEKNIKEFLQSFVHIVQMFINTS | 59 |

| **3F815 TL-2 (IL-15 (2 copies) connected to each C terminus of the light chain (2 copies) of anti-TIGIT antibody through linker V-2)** | | |
|---|---|---|
| Heavy-chain strand (TIGIT mAb heavy chain (IgG1)) | EVQLQQSGPELVKPGASMKISCKASGYSFTDYIMNWVK QSHGKNLEWIGLSIPYNGGTSYNQKFEGKATLTVDKSSS TAYMELLSLTSEDSAVYYCARGIKGYFAMDYWGQGTSV TVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKRVEPKSCDKTHTCPPCPAP ELLGGPSVFLFPPKPKDTLMISRTPEVTCVVVDVSHEDPE VKFNWYVDGVEVHNAKTKPREEQYNSTYRVVSVLTVL HQDWLNGKEYKCKVSNKALPAPIEKTISKAKGQPREPQV YTLPPSREEMTKNQVSLTCLVKGFYPSDIAVEWESNGQP ENNYKTTPPVLDSDGSFFLYSKLTVDKSRWQQGNVFSCS VMHEALHNHYTQKSLSLSPGK | 57 |
| Light-chain strand ([TIGIT mAb light chain(kappk)]-[linker(V-2)]-[IL-15]) | DVVVTQTPLSLPVSFGDQVSISCRSSQSLVNSFGKTYLSW FLHKPGQSPQLIMYGVSNRFSGVPDRFSGSGSGTDFTLKI STIKPEDLGMYYCLQGTHQPWTFGGGTKLEIKRTVAAPS VFIFPPSDEQLKSGTASVVCLLNNFYPREAKVQWKVDNA LQSGNSQESVTEQDSKDSTYSLSSTLTLSKADYEKHKVY ACEVTHQGLSSPVTKSFNRGECEAAAKEAAAKEAAAKN WVNVISDLKKIEDLIQSMHIDATLYTESDVHPSCKVTAM KCFLLELQVISLESGDASIHDTVENLIILANNSLSSNGNVT ESGCKECEELEEKNIKEFLQSFVHIVQMFINTS | 60 |

| **3F815 TL-3 (IL-15 (2 copies) connected to each C terminus of the heavy chain (2 copies) of anti-TIGIT antibody through linker V-1)** | | |
|---|---|---|
| Heavy-chain strand ([TIGIT mAb heavy chain(IgG1)]-[linker(V-1)]-[IL-15]) | EVQLQQSGPELVKPGASMKISCKASGYSFTDYIMNWVK QSHGKNLEWIGLSIPYNGGTSYNQKFEGKATLTVDKSSS TAYMELLSLTSEDSAVYYCARGIKGYFAMDYWGQGTSV TVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKRVEPKSCDKTHTCPPCPAP ELLGGPSVFLFPPKPKDTLMISRTPEVTCVVVDVSHEDPE VKFNWYVDGVEVHNAKTKPREEQYNSTYRVVSVLTVL HQDWLNGKEYKCKVSNKALPAPIEKTISKAKGQPREPQV YTLPPSREEMTKNQVSLTCLVKGFYPSDIAVEWESNGQP ENNYKTTPPVLDSDGSFFLYSKLTVDKSRWQQGNVFSCS VMHEALHNHYTQKSLSLSPGKGGGGSGGGGSGGGGSN WVNVISDLKKIEDLIQSMHIDATLYTESDVHPSCKVTAM KCFLLELQVISLESGDASIHDTVENLIILANNSLSSNGNVT ESGCKECEELEEKNIKEFLQSFVHIVQMFINTS | 61 |
| Light-chain strand (TIGIT mAb light chain(kappk)) | DVVVTQTPLSLPVSFGDQVSISCRSSQSLVNSFGKTYLSW FLHKPGQSPQLIMYGVSNRFSGVPDRFSGSGSGTDFTLKI STIKPEDLGMYYCLQGTHQPWTFGGGTKLEIKRTVAAPS VFIFPPSDEQLKSGTASVVCLLNNFYPREAKVQWKVDNA LQSGNSQESVTEQDSKDSTYSLSSTLTLSKADYEKHKVY ACEVTHQGLSSPVTKSFNRGEC | 58 |

| **3F815 TL-4 (IL-15 (2 copies) connected to each C terminus of the heavy chain (2 copies) of anti-TIGIT antibody through linker V-2)** | | |
|---|---|---|
| Heavy-chain strand ([TIGIT mAb heavy chain (IgG1)]-[linker (V-2)]-[IL-15]) | EVQLQQSGPELVKPGASMKISCKASGYSFTDYIMNWVK QSHGKNLEWIGLSIPYNGGTSYNQKFEGKATLTVDKSSS TAYMELLSLTSEDSAVYYCARGIKGYFAMDYWGQGTSV TVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKRVEPKSCDKTHTCPPCPAP ELLGGPSVFLFPPKPKDTLMISRTPEVTCVVVDVSHEDPE VKFNWYVDGVEVHNAKTKPREEQYNSTYRVVSVLTVL HQDWLNGKEYKCKVSNKALPAPIEKTISKAKGQPREPQV YTLPPSREEMTKNQVSLTCLVKGFYPSDIAVEWESNGQP ENNYKTTPPVLDSDGSFFLYSKLTVDKSRWQQGNVFSCS VMHEALHNHYTQKSLSLSPGKEAAAKEAAAKEAAAKN WVNVISDLKKIEDLIQSMHIDATLYTESDVHPSCKVTAM KCFLLELQVISLESGDASIHDTVENLIILANNSLSSNGNVT ESGCKECEELEEKNIKEFLQSFVHIVQMFINTS | 62 |
| Light-chain strand (TIGIT mAb light chain (kappk)) | DVVVTQTPLSLPVSFGDQVSISCRSSQSLVNSFGKTYLSW FLHKPGQSPQLIMYGVSNRFSGVPDRFSGSGSGTDFTLKI STIKPEDLGMYYCLQGTHQPWTFGGGTKLEIKRTVAAPS VFIFPPSDEQLKSGTASVVCLLNNFYPREAKVQWKVDNA LQSGNSQESVTEQDSKDSTYSLSSTLTLSKADYEKHKVY ACEVTHQGLSSPVTKSFNRGEC | 58 |
| **3F815 TL-5 (IL-15 (2 copies) connected to each C terminus of the heavy chain (2 copies) of anti-TIGIT antibody without linker)** | | |
| Heavy-chain strand ([TIGIT mAb heavy chain (IgG1)]- -[IL-15]) | EVQLQQSGPELVKPGASMKISCKASGYSFTDYIMNWVK QSHGKNLEWIGLSIPYNGGTSYNQKFEGKATLTVDKSSS TAYMELLSLTSEDSAVYYCARGIKGYFAMDYWGQGTSV TVSSASTKGPSVFPLAPSSKSTSGGTAALGCLVKDYFPEP VTVSWNSGALTSGVHTFPAVLQSSGLYSLSSVVTVPSSSL GTQTYICNVNHKPSNTKVDKRVEPKSCDKTHTCPPCPAP ELLGGPSVFLFPPKPKDTLMISRTPEVTCVVVDVSHEDPE VKFNWYVDGVEVHNAKTKPREEQYNSTYRVVSVLTVL HQDWLNGKEYKCKVSNKALPAPIEKTISKAKGQPREPQV YTLPPSREEMTKNQVSLTCLVKGFYPSDIAVEWESNGQP ENNYKTTPPVLDSDGSFFLYSKLTVDKSRWQQGNVFSCS VMHEALHNHYTQKSLSLSPGKNWVNVISDLKKIEDLIQS MHIDATLYTESDVHPSCKVTAMKCFLLELQVISLESGDA SIHDTVENLIILANNSLSSNGNVTESGCKECEELEEKNIKE FLQSFVHIVQMFINTS | 63 |
| light-chain strand (TIGIT mAb light chain (kappk)) | DVVVTQTPLSLPVSFGDQVSISCRSSQSLVNSFGKTYLSW FLHKPGQSPQLIMYGVSNRFSGVPDRFSGSGSGTDFTLKI STIKPEDLGMYYCLQGTHQPWTFGGGTKLEIKRTVAAPS VFIFPPSDEQLKSGTASVVCLLNNFYPREAKVQWKVDNA LQSGNSQESVTEQDSKDSTYSLSSTLTLSKADYEKHKVY ACEVTHQGLSSPVTKSFNRGEC | 58 |

The definitions related to the linkers between the anti-TIGIT antibody and IL-15 in the fusion proteins described in Tables 4 to 6 and the following examples are summarized as follows:
TL-1: Linker V-1 attached to the C-terminus of the light chain of the anti-TIGIT antibody;
TL-2: Linker V-2 attached to the C-terminus of the light chain of the anti-TIGIT antibody;
TL-3: Linker V-1 attached to the C-terminus of the heavy chain (Fc) of the anti-TIGIT antibody;
TL-4: Linker V-2 attached to the C-terminus of the heavy chain (Fc) of the anti-TIGIT antibody;
TL-5: No linker between the heavy chain (Fc) of the anti-TIGIT antibody and IL-15.

### EXAMPLE 2. Measurement of Binding Affinity of the Fusion Protein to Human TIGIT (ELISA)

An ELISA plate was coated with recombinant human TIGIT protein (Acrobiosystems, China) at a concentration of 1 µg/ml dissolved in PBS (Phosphate-Buffered Saline) and incubated overnight at 4°C. The plate was blocked with blocking buffer (3% (v/v) skim milk in PBS) for 1 hour, and serially diluted anti-TIGIT monoclonal antibody (7A6) and the fusion protein ([7A6]-[TL-2]-[IL-15]; denoted as '7A615TL-2') (starting concentration: 61.097 nM; serial 1:3 dilutions) were added to the coated plate. After incubation, anti-mouse/human IgG-HRP antibody (Abcam; 1:10000 dilution) was added to each well. The plate was developed with TMB solution and stopped with 1N HCl. The OD value was read at 450 nm using the GloMax^{®} Explorer Fully Loaded.

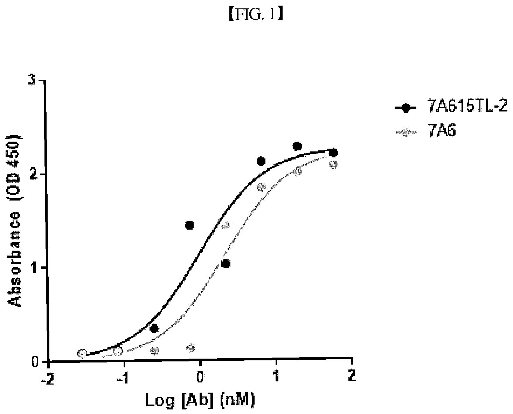

The results obtained are shown in FIG. 1. As shown in the results, the 7A615TL-2 fusion protein bound to human TIGIT with a binding affinity similar to that of the parent antibody (anti-TIGIT monoclonal antibody 7A6). This result indicates that the fusion of IL-15 with the anti-TIGIT antibody did not affect the antigen-binding site of the antibody, maintaining all the functional properties of the parent antibody.

### EXAMPLE 3. Increased Cytokine Secretion of Human PBMCs by the Fusion Protein

To measure cytokine production in human CD3+ T cells and CD56+ NK cells, 500,000 human-derived PBMCs (Stemcell Technologies) were incubated with anti-CD3 monoclonal antibody (BD Biosciences, 0.2 µg/ml; or anti-CD4 antibody or anti-CD8 antibody) and anti-CD28 monoclonal antibody (BD Biosciences, 1 µg/ml) in the presence of anti-TIGIT 7A6 antibody (10 µg/ml) or the fusion protein (7A615TL-2, 68.79 nM). The control group consisted of cells not treated with the anti-TIGIT antibody or 7A615TL-2. After 4 hours of incubation at 37°C and 5% CO₂, Brefeldin A (10 µg/ml) was added to inhibit cytokine secretion, and the cells were incubated for an additional 15 hours, for a total incubation time of 19 hours.

To analyze the proportion of cytokine-producing cells, the cells were washed twice by spinning down at 400 x g for 10 minutes at 4°C, and then stained as follows: The cells were stained with Zombie Aqua fixable dead cell dye solution (Biolegend) at room temperature for 20 minutes, then washed by spinning down at 400 x g for 10 minutes. The cells were subsequently labeled with fluorophore-conjugated antibodies against surface markers for CD4+ T cells, CD8+ T cells, or CD56+ NK cells for 30 minutes on ice. All anti-CD3, anti-CD4, anti-CD8, and anti-CD56 antibodies were obtained from Biolegend.

The cells were fixed and permeabilized using the eBioscience^{™} Foxp3/Transcription Factor Fixation/Permeabilization Concentrate and Diluent kit (eBiosciences) according to the manufacturer's instructions. The cells were then stained with fluorophore-conjugated antibodies against intracellular IFN-γ and TNFα (Biolegend) for 30 minutes at room temperature. After washing the cells, fluorescence for surface marker labeling and cytokines was detected using a flow cytometer (CytoFLEX, Beckman Coulter). Data were analyzed using FlowJo software (BD Biosciences) and visualized using GraphPad Prism 9 software (Dotmatics).

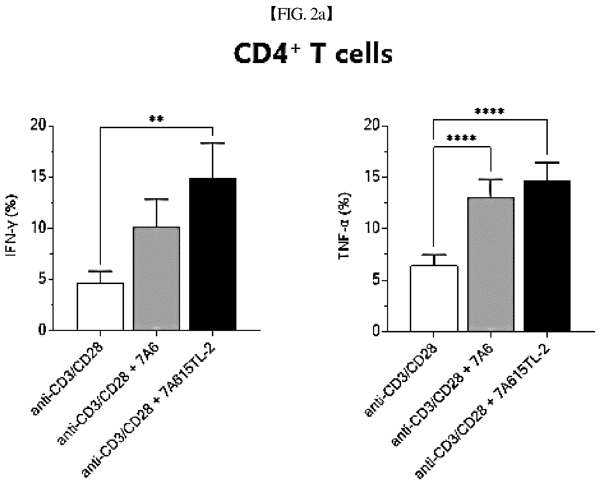

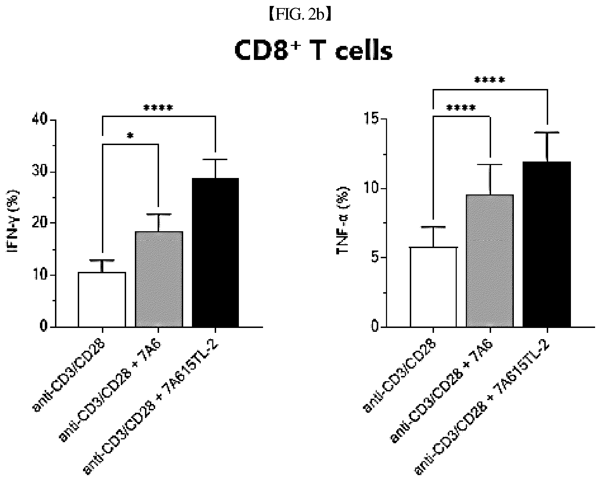

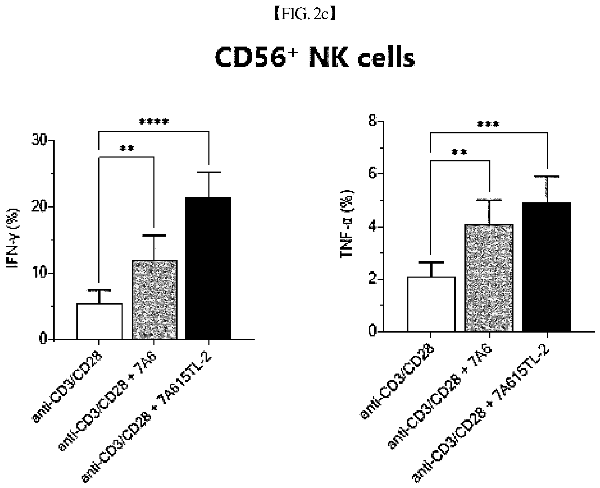

The results are shown in FIG. 2a (CD4+ T cells), FIG. 2b (CD8+ T cells), and FIG. 2c (CD56+ NK cells). As shown in FIGS. 2a to 2c, the fusion protein provided herein increased the production of Th1/Tc1 cytokines (IFN-gamma) and TNF-alpha in T cells and NK cells, thereby enhancing the immune response. The results suggest that 7A615 exhibits a stronger and dose-dependent effect on enhancing effector T cell and NK cell function compared to the anti-TIGIT antibody.

### EXAMPLE 4. Increased Cytokine Expression by Fusion Protein

Human peripheral blood mononuclear cells (PBMCs) derived from healthy donors were plated at 0.15 - 0.16 x 10⁶ cells in round-bottom 96-well plates and cultured at 37°C and 5% CO₂ for 44 hours in the presence of anti-CD3 antibody (Biolegend) at 1 µg/ml. The 7A615TL-2 fusion protein and the parent antibody (7A6) were added to the culture medium at the same concentration (68.79 nM). The culture volume was 250 µl per well. After incubation, the resulting culture was centrifuged at 660 x g for 10 minutes at 4°C. The supernatants were collected in 1.5 ml microcentrifuge tubes and stored at -80°C.

Cytokine levels were measured using the Bio-Plex cytokine assay (4-plex; #PPX-04, Invitrogen). The 4-plex cytokine analysis was performed for the following four human cytokines: IFN-γ, IL-2, TNF-α, and IL-9. All analyses were conducted according to the manufacturer's instructions, and the results were read using a Bio-Plex 200 array reader (Bio-Rad).

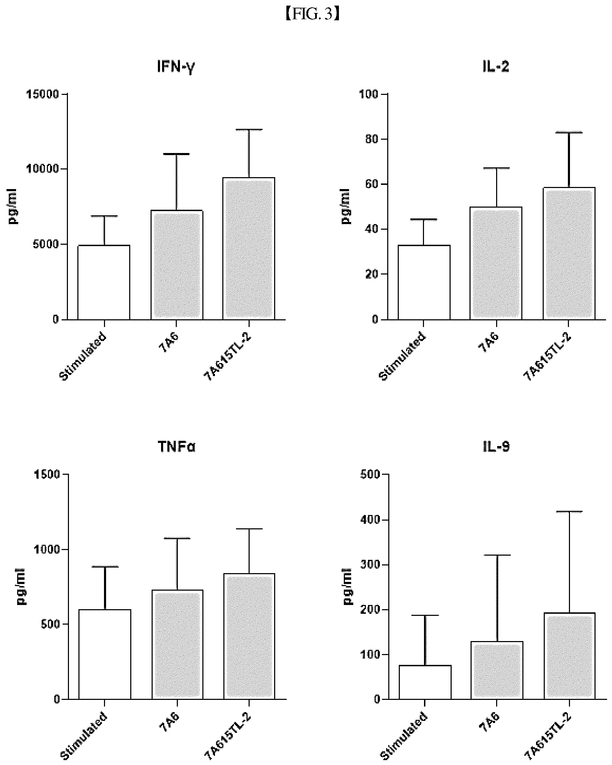

The obtained results are shown in FIG. 3. In the in vitro culture of human PBMCs activated by anti-CD3 monoclonal antibody to stimulate T cells, co-stimulation of T cells with the fusion protein (7A615TL-2) provided herein resulted in higher levels of cytokine (IFN-γ, IL-2, TNF-α, and IL-9) expression compared to co-stimulation with the parent antibody (7A6).

### EXAMPLE 5. Induction of STAT5 Phosphorylation by Fusion Protein

The cells (Human PBMCs) were thawed and resuspended in R10 medium (RPMI1640 medium (Gibco) containing 10% (v/v) FBS (Gibco), Penicillin-Streptomycin (Sigma), and L-glutamine (Sigma)) at a concentration of 10⁶ cells/mL. A total of 100 µl of the cell suspension was seeded into each well (equaling 1x10⁶ cells/well). The 7A615TL-2 fusion protein was diluted to 68.7 nM, and starting from this concentration, serial 10-fold dilutions were made down to 69 fM. The cells and the fusion protein were mixed thoroughly by pipetting and incubated at 37°C for 15 minutes. After incubation, centrifugation at 500 x g for 5 minutes pelletized the cells. The supernatant was discarded, and the pellet was resuspended by vortexing. The cells were then resuspended in PBS containing viability marker (eBioScience, 1:500), anti-CD3 antibody (Biolegend, 1:50), and anti-CD8 antibody (Biolegend, 1:50). The cells were stained at room temperature for 10 minutes, followed by centrifugation at 500 x g for 5 minutes to harvest the cells as a pellet, and the supernatant was discarded. The pellet was vortexed to break up the cells. The cells were then resuspended in 200 µL of Fixation Buffer (Biolegend) pre-warmed to 37°C and incubated at room temperature for 15 minutes. The cell suspension was centrifuged at 500 x g for 5 minutes to pelletize the cells, and the supernatant was discarded. The cell pellet was vortexed to break up the cells, and the cells were then resuspended in 200 µL of ice-cold True-Nuclear 1X Perm Buffer (Biolegend). The cells were stored at -20°C for 1 hour, followed by centrifugation at 500 x g for 5 minutes to harvest the cells as a pellet. The supernatant was discarded, and the pellet was vortexed to break up the cells. The cells were then resuspended in staining buffer containing anti-pSTAT5 antibody (Biolegend, 1:25) and incubated at room temperature for 15 minutes. The cell suspension was centrifuged at 500 x g for 5 minutes and the supernatant was discarded. The resulting pellet was vortexed and resuspended in PBS. The cells were then centrifuged again as described above, and finally, the cells were resuspended in 100 µL of PBS, and data were acquired using flow cytometry (CytoFlex, BeckmanCoulter). Data were analyzed using FlowJo (BD, USA).

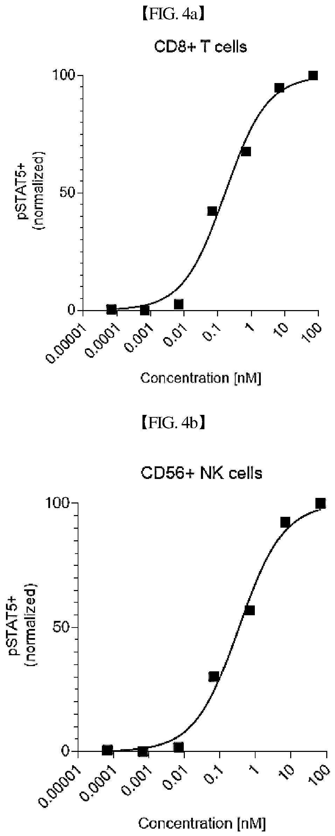

The results obtained are shown in FIG. 4a (CD8+ T cells) and FIG. 4b (NK cells). IL-15 linked to the anti-TIGIT antibody induced STAT5 phosphorylation in both CD8+ T cells and NK cells. In primary cells, receptor heterodimerization must induce signal transduction across the membrane. As for IL-15 receptor complex signaling, its measurement was conducted by STAT5 phosphorylation. Stimulation of CD8+ T cells and NK cells with the fusion protein provided herein induced high levels of STAT5 phosphorylation, demonstrating that IL-15 in the fusion protein retains its ability to induce strong signaling.

### EXAMPLE 6. Long-term Cytokine Production Sustained by Fusion Protein

The fusion protein provided herein was observed to stimulate immune cells over a long period, inducing sustained cytokine production.

More specifically, the ability of the 7A615TL-2 fusion protein to induce long-term signaling was tested.

To this end, PBMCs from healthy human donors were seeded at a density of 250,000 cells per well in a 96-well U-bottom plate containing R10 medium (RPMI1640 medium (Gibco) with 10% (v/v) FBS (Gibco), Penicillin-Streptomycin (Sigma), and L-glutamine (Sigma)). The cells were stimulated with IL-15 or the 7A615TL-2 fusion protein at a concentration of 378.6 pM for 1 hour at 37°C and 5% CO₂, then centrifuged at 400 x g for 3 minutes, and washed five times with R10 medium. The cells were then left to rest for 24 hours at 37°C and 5% CO₂.

After the 24-hour rest period, the cells were stimulated with anti-CD3 antibody (CytoStim, 1:500; Miltenyi) and incubated overnight at 37°C and 5% CO₂. Two hours after adding CytoStim as a stimulator, Brefeldin A (Biolegend, 1:1000) and Monensin (Biolegend, 1:1000) were added. The cells were centrifuged at 400 x g for 3 minutes and washed with PBS, then resuspended in 50 µl of PBS containing Fixable Viability dye eFluor 780 (Thermo Fisher, 1:500). The cells were incubated in the dark at room temperature for 10 minutes, then centrifuged at 400 x g for 3 minutes and washed with 100 µl of PBS. After another centrifugation at 400 x g for 3 minutes, the cells were resuspended in 50 µl of Fixation Buffer (Biolegend) and incubated in the dark at room temperature for 30 minutes. The cells were then centrifuged again at 400 x g for 3 minutes, washed with 100 µl of Perm Buffer (Biolegend, diluted to a 1x solution in water), and resuspended in 200 µl of Perm Buffer. The cells were incubated in the dark at room temperature for 1 hour in the Perm Buffer, centrifuged at 400 x g for 5 minutes, and the supernatant was discarded. The cell pellet thus obtained was resuspended in 50 µl of Perm Buffer containing the following antibodies: anti-CD4-AlexaFluor700 (1:50), anti-CD8-PerCP (1:50), anti-CD56-BV421 (1:50), anti-IL-2-PE (1:50), and anti-IFN-γ-F1TC (1:50). All antibodies were obtained from Biolegend. The cells were centrifuged at 400 x g, the supernatant was discarded, and the cell pellet was resuspended in 100 µl of PBS for analysis using a CytoFlex flow cytometer.

FCS files were generated using CytExpert Software (Beckman Coulter) and analyzed using FlowJo v10.8.1. Graph generation and statistical analyses were performed using GraphPad Prism 9. Statistical significance was assessed by One-Way ANOVA corrected for multiple comparisons with a Dunnett's test (n=8), * P≤0.05, ** P≤0.01, *** P≤0.001, **** P≤0.0001.

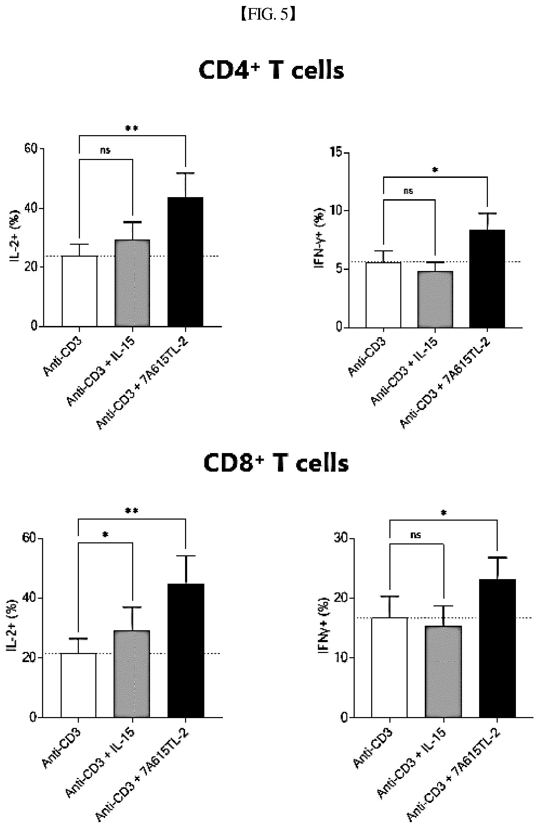

The results obtained are depicted in FIG. 5. As shown in FIG. 5, stimulation with the fusion protein followed by a 24-hour rest period resulted in a significant increase in cytokine (IL-2, IFN-γ) production in both CD4+ and CD8+ T cells. However, this increase was generally not observed when cells were stimulated with IL-15 alone. These results demonstrate that the anti-TIGIT antibody component of the fusion protein promotes sustained signaling on the surface of T cells, and the fusion protein retains long-term activity.

### EXAMPLE 7. Fusion Protein-Mediated Depletion of Treg Cells

Frozen human peripheral blood mononuclear cells (PBMCs) from Birmingham Hospital were thawed and left to rest for 16 hours at 37°C and 5% CO₂. The next day, Treg cells and NK cells were both isolated by magnetic bead isolation (Miltenyi Biotec). The cells were counted and seeded at a ratio of 10 NK cells per 1 Treg cell in a 96 U-bottom plate. The 7A615TL-2 fusion protein was added to the wells at a concentration of 10 µg/mL. The co-cultured cells were incubated for 24 hours at 37°C and 5% CO₂. After incubation, the co-cultured cells were centrifuged at 500 x g for 5 minutes to pellet the cells. The supernatant was discarded, and the pellet was vortexed to break up the cells. The cells were resuspended in phosphate-buffered saline (PBS), followed by centrifugation at 500 x g for 5 minutes to harvest the cells as a pellet again. The cells were then resuspended in a staining buffer containing viability marker (eBioScience, 1:500), anti-CD3 antibody (Biolegend, 1:50), anti-CD4 antibody (Biolegend, 1:50), anti-CD127 antibody (Biolegend, 1:50), anti-CD25 antibody (Biolegend, 1:50), and anti-CD56 antibody (Biolegend, 1:50) and incubated in the dark for 10 minutes. The cells were then washed with 200 µl of PBS. Finally, the cells were resuspended in 100 µl of PBS, and data were obtained using flow cytometry (CytoFlex, Beckman Coulter). The data were analyzed using FlowJo (BD, USA).

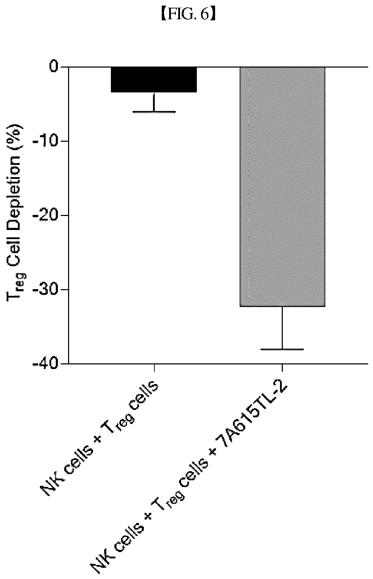

The results obtained are shown in FIG. 6. As seen in FIG. 6, the fusion protein provided in this application demonstrates a strong ability to deplete Treg cells during the co-culture with NK cells.

### EXAMPLE 8. Proliferation of T Cells, NKT Cells, and NK Cells by Fusion Protein Treatment

PBMCs obtained from four healthy human donors were counted and centrifuged at 400 x g for 3 minutes. The PBMCs were resuspended at a concentration of 5 x 10⁶ cells/ml in PBS containing CFSE dye (Biolegend, 1:2000 dilution). After this step, the cells were incubated for 20 minutes in a 37°C incubator, protected from direct light. The tubes were then filled with R10 medium, and the cells were incubated for an additional 10 minutes at 37°C. After centrifuging the cells (PBMCs) at 400 x g for 3 minutes, they were washed three times with R10 medium, counted, and seeded at 500,000 cells in 100 µl of R10 medium. The cells were then stimulated with 7A615TL-2 fusion protein at a concentration of 3876 pM or soluble IL-15 (Peprotech), and incubated for 7 days at 37°C and 5% CO₂. After incubation, the cells were centrifuged at 400 x g for 3 minutes and stained with a 50 µl PBS solution containing an antibody cocktail (anti-CD3 APC (Biolegend; 1:50), anti-CD4-AF700 (Biolegend, 1:50), anti-CD56 BV421 (Biolegend, 1:50), and Fixable Viability Dye eFluor 780 (eBioscience; 65-0865-18)). The cells were incubated at room temperature for 10 minutes, centrifuged at 400 x g for 3 minutes, and washed with 100 µl of PBS. The cells were then resuspended in 100 µl of PBS and analyzed using a CytoFlex Flow Cytometer (Beckman Coulter). The obtained data were analyzed using FlowJo v10.8.1. The gating strategy defined T cells as CD3+CD56-, NKT cells as CD3+CD56+, and NK cells as CD3-CD56+. As cells divide, the level of CFSE dye diminishes, so all cells with lower CFSE expression than the starting population were gated for total proliferation. Unstimulated controls from each donor were used to set the threshold. Graph generation and statistical analyses were performed using GraphPad Prism 9. Statistical significance was assessed by One-Way ANOVA corrected for multiple comparisons with Dunnett's test (n=8), * P≤0.05, ** P≤0.01, *** P≤0.001, **** P≤0.0001.

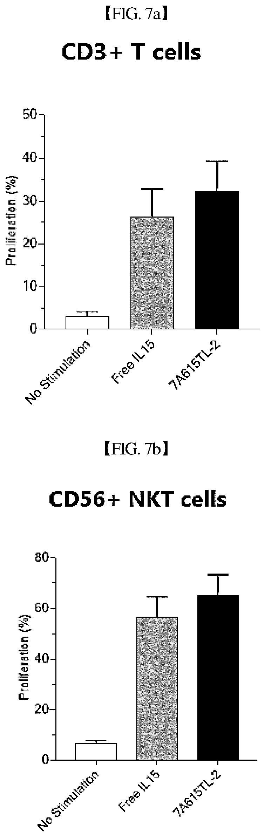

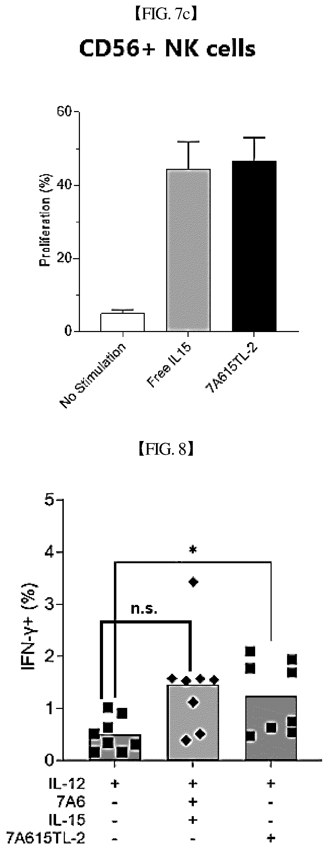

The results obtained are shown in FIG. 7a (T cells), FIG. 7b (NKT cells), and FIG. 7c (NK cells). In this experiment, PBMC proliferation was measured using CFSE dye. All three cell populations (T cells, NKT cells, NK cells) showed significant proliferation after stimulation with either 7A615TL-2 fusion protein or free IL-15, with slightly higher proliferation observed in cells stimulated with the 7A615TL-2 fusion protein. This suggests that the 7A615TL-2 fusion protein is at least as effective as free IL-15 in stimulating the proliferation of T cells, NKT cells, and NK cells. These results demonstrate that the IL-15 component within the fusion protein retains its ability to stimulate immune cell proliferation.

### EXAMPLE 9. Effect of Fusion Protein on Cytokine Expression in NK Cells

An examination was conducted to see whether immune cell stimulation by the fusion protein provided herein could induce prolonged signaling.

To this end, PBMCs obtained from healthy human donors were seeded at a density of 500,000 cells per well in a 96-well U-bottom plate in R10 medium (RPMI1640 medium (Gibco) containing 10% (v/v) FBS (Gibco), Penicillin-Streptomycin (Sigma), and L-glutamine (Sigma)). The cells were stimulated with IL-15 or the 7A615TL-2 fusion protein at a concentration of 378.6 pM for 1 hour at 37°C and 5% CO₂. The cells were then centrifuged at 400 x g for 3 minutes and washed five times with R10 medium. After washing, the cells were left to rest for 24 hours at 37°C and 5% CO₂.

Following the 24-hour resting, the cells were stimulated with IL-12 (Peprotech) at a concentration of 50 ng/ml and incubated overnight at 37°C and 5% CO₂. After 2 hours of stimulation with CytoStim (Miltenyi Biotec), Brefeldin A (Biolegend, 1:1000) and Monensin (Biolegend, 1:1000) were added. The cells were centrifuged at 400 x g for 3 minutes, washed with PBS, and resuspended in 50 µl PBS containing Fixable Viability Dye eFluor 780 (Thermo Fisher, 1:500). The cells were incubated at room temperature for 10 minutes in the dark, centrifuged at 400 x g for 3 minutes, and washed with 100 µl of PBS. The cells were centrifuged again at 400 x g for 3 minutes, resuspended in 50 µl of fixation buffer (Biolegend), and incubated in the dark at room temperature for 30 minutes. The cells were washed with 100 µl of Perm buffer (Biolegend; diluted to a 1x solution in water) and incubated in the dark at room temperature in Perm buffer for 1 hour. After washing and centrifugation, the cells were resuspended in 50 µl of Perm buffer containing the following antibodies: CD3-APC (1:50), CD4-AlexaFluor700 (1:50), CD8-PerCP (1:50), CD56-BV421 (1:50), and IFN-γ-FITC (1:50). All antibodies were obtained from Biolegend. The cells were then centrifuged at 400 x g, resuspended in 100 µl of PBS, and analyzed using a CytoFlex flow cytometer (Beckman Coulter).

Data were processed using CytExpert Software (Beckman Coulter) to generate FCS files and analyzed with FlowJo v10.8.1. Graphs and statistical analyses were performed using GraphPad Prism 9. Statistical significance was assessed by One-Way ANOVA corrected for multiple comparisons with Dunnett's test (n=8), with significance levels indicated as * P≤0.05, ** P≤0.01, *** P≤0.001, **** P≤0.0001.

The results are shown in FIG. 8. As shown, stimulation with the 7A615TL-2 fusion protein significantly induced high levels of IFN-γ expression in NK cells after a 24-hour rest period, followed by re-stimulation with IL-12. In contrast, no significant increase in IFN-γ production was observed in the combination treatment of the 7A6 antibody and IL-15 compared to the control group (fusion protein or combination treatment not applied) (n.s. = non-significant). These results demonstrate that the fusion protein in this application can provide sustained signaling to induce prolonged IFN-γ production.

### EXAMPLE 10. Cytotoxicity of Fusion Protein (Lung Cancer Cells; A549)

Frozen human PBMCs were thawed, washed, and resuspended in complete medium (RPMI Merk, UK, plus 10% (v/v) FCS Merk, UK). The cells were stimulated with IL-15 (Peprotech) or 7A615TL-2 fusion protein at a concentration of 68.7nM for 1 hour. The cells were stimulated at a density of 5x10⁶ cells/ml. The cells were washed five times by centrifugation at 1500 rpm for 3 minutes in complete medium (2 ml each). After washing, the cells were resuspended at a density of 2x10⁶ cells/ml (100,000 cells in 50 µl) and transferred to a 96-well U-bottom plate, where they were left to rest for 24 hours.

On the day of analysis, adherent A549-GFP target cells (A549-GFP (lung) MERK (ECACC) cat# 86012804-1VL) were washed with PBS (phosphate-buffered saline) and incubated with Trypsin/EDTA (0.05%, Gibco, Fisher UK) for 5 minutes at 37°C and 5% CO₂. The A549 cells were harvested, centrifuged at 500 x g for 5 minutes, and pelletized. The supernatant was discarded, and the A549-GFP cells were resuspended in complete medium (RPMI Merk, UK, and 10% FCS Merk, UK) and seeded into a 96 flat-bottom well plate at a density of 5 x 10³ cells/well in 50 µl. The cells were allowed to adhere for 3 hours at 37°C and 5% CO₂. The prepared PBMCs were then added to the adhered A549-GFP cells at a density of 1x10⁵ cells/ml in 50 µl/well. Anti-CD3 antibody (Biolegend) was added at a concentration of 10 ng/ml. The cell death marker DRAQ7 (Biolegend) was diluted to 1/200, resulting in a final concentration of 1.5 µM, and added to each well. The co-culture was incubated in the IncuCyte (Sartorius, UK) at 37°C and 5% CO₂. Images were taken every 2 hours for 5 days, and the results were analyzed using IncuCyte's native software.

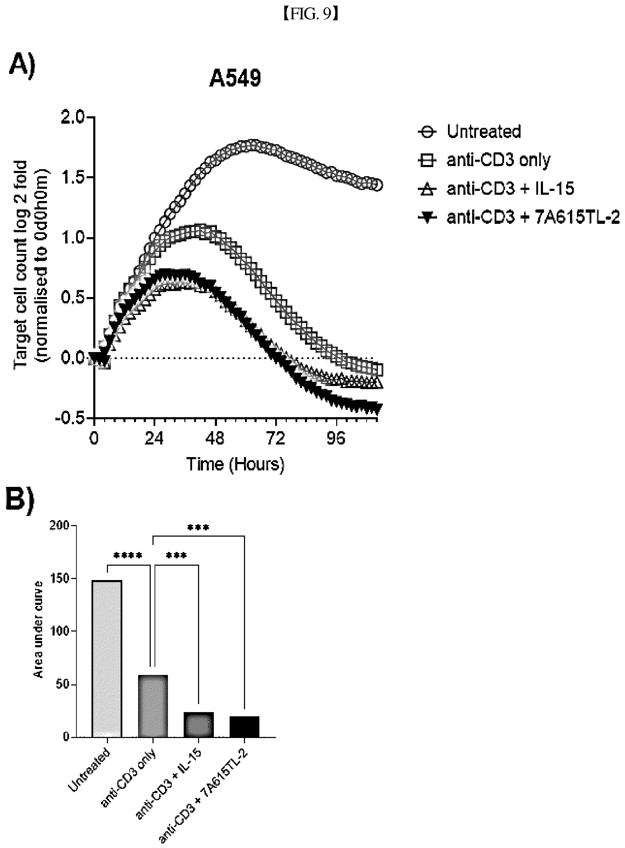

The obtained results are shown in FIG. 9 [A) shows the log2 fold change in A549 cell numbers over time, and B) shows the area under the curve from A)]. Compared to untreated, anti-CD3 antibody-treated PBMCs or PBMCs treated with anti-CD3 antibody and IL-15, the PBMCs activated by the 7A615TL-2 fusion protein of this application showed significantly higher levels of cytotoxicity against the A549-GFP target cell line. Notably, these PBMCs exhibited sustained cytotoxicity over a prolonged period (up to 72 hours) compared to PBMCs treated with anti-CD3 antibody and IL-15.

### EXAMPLE 12. Cytotoxicity of Fusion Protein (Breast Cancer Cells; MCF-7)

Frozen human PBMCs were thawed. NK cells were isolated using magnetic bead isolation (Miltney UK). The isolated cells were pre-activated by incubation for 24 hours at 37°C and 5% CO₂ in the presence or absence of 10 µg/ml of the 7A615TL-2 fusion protein or Pembrolizumab (Invivogen). On the day of the analysis, adherent MCF-7-GFP target cells (MCF7-GFP (breast); MacroPhox Ltd) were washed with PBS and lifted by incubation with trypsin/EDTA (0.05%, Gibco, Fisher UK) at 37°C and 5% CO₂ for 5 minutes. The MCF-7 cells were harvested and centrifuged at 500 x g for 5 minutes to form a pellet. The supernatant was discarded, and the MCF-7-GFP cells were resuspended in complete medium (RPMI Merk, UK, plus 10%(v/v) FCS Merk, UK), and then seeded at a density of 1 x 10⁴ cells/well in 50 µl in a 96-well flat-bottom plate. The cells were allowed to adhere by resting at 37°C and 5% CO₂ for 3 hours. The pre-activated NK cells were then added to the adhered MCF-7-GFP cells at a density of 1 x 10⁵ cells/ml in 50 µl/well. Additionally, 10 µg/ml of the 7A615TL-2 fusion protein or Pembrolizumab (Invivogen) was added to each well. The co-cultures were incubated in an IncuCyte (Sartorius, UK) at 37°C and 5% CO₂. Four images were taken every 2 hours for 6 days. The analysis was performed using the native software of the IncuCyte.

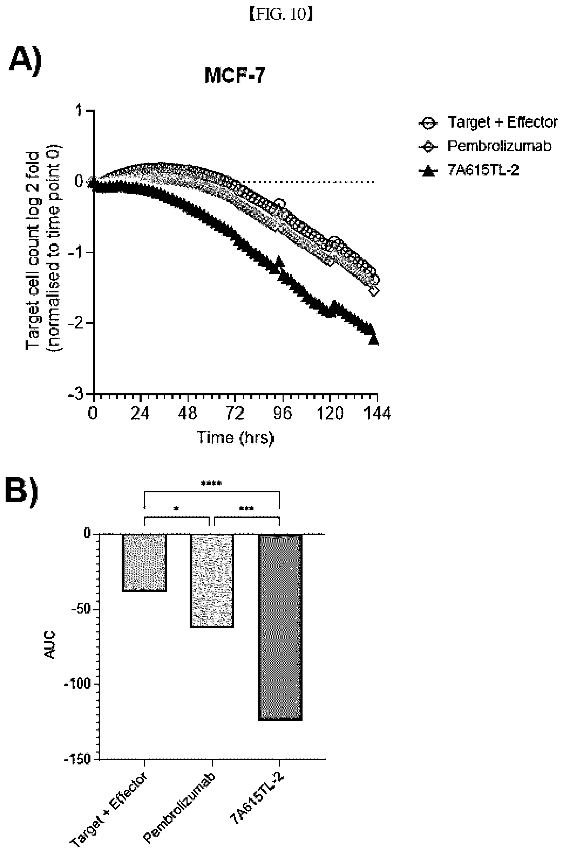

The results obtained are shown in FIG. 10 [A) shows the log2 fold change of the number of MCF-7 cells over time, B) shows the area under the curve (AUC) of A)]. Compared to the untreated group or the Pembrolizumab-treated group, NK cells treated with the 7A615TL-2 fusion protein showed a significantly higher level of cytotoxicity towards the MCF-7-GFP target cell line.

## Claims

1. A fusion protein, comprising:
an anti-TIGIT antibody or an antigen-binding fragment thereof; and
interleukin-15 (IL-15),
wherein the anti-TIGIT antibody or the antigen-binding fragment thereof comprises
a CDR-H1 including the amino acid sequence of SEQ ID NO: 1, 2, or 3,
a CDR-H2 including the amino acid sequence of SEQ ID NO: 4, 5, or 6,
a CDR-H3 including the amino acid sequence of SEQ ID NO: 7, 8, or 9,
a CDR-L1 including the amino acid sequence of SEQ ID NO: 10, 13, or 14,
a CDR-L2 including the amino acid sequence of SEQ ID NO: 15, 16, or 17, and
a CDR-L3 including the amino acid sequence of SEQ ID NO: 18, 19, or 20.

2. The fusion protein of Claim 1, wherein the anti-TIGIT antibody or the antigen-binding fragment thereof comprises:
a CDR-H1 including the amino acid sequence of SEQ ID NO: 1, a CDR-H2 including the amino acid sequence of SEQ ID NO: 4, a CDR-H3 including the amino acid sequence of SEQ ID NO: 7, a CDR-L1 including the amino acid sequence of SEQ ID NO: 11 or 12, a CDR-L2 including the amino acid sequence of SEQ ID NO: 15, and a CDR-L3 including the amino acid sequence of SEQ ID NO: 18;
a CDR-H1 including the amino acid sequence of SEQ ID NO: 2, a CDR-H2 including the amino acid sequence of SEQ ID NO: 5, a CDR-H3 including the amino acid sequence of SEQ ID NO: 8, a CDR-L1 including the amino acid sequence of SEQ ID NO: 13, a CDR-L2 including the amino acid sequence of SEQ ID NO: 16, and a CDR-L3 including the amino acid sequence of SEQ ID NO: 19; or
a CDR-H1 including the amino acid sequence of SEQ ID NO: 3, a CDR-H2 including the amino acid sequence of SEQ ID NO: 6, a CDR-H3 including the amino acid sequence of SEQ ID NO: 9, a CDR-L1 including the amino acid sequence of SEQ ID NO: 14, a CDR-L2 including the amino acid sequence of SEQ ID NO: 17, and a CDR-L3 including the amino acid sequence of SEQ ID NO: 20.

3. The fusion protein of Claim 1, wherein the anti-TIGIT antibody or the antigen-binding fragment thereof comprises:
a heavy chain variable region including the amino acid sequence of SEQ ID NO: 21, 22, 23, 24, 25, 26, 27, or 28; and
a light chain variable region including the amino acid sequence of SEQ ID NO: 29, 30, 31, 32, 33, 34, 35, or 36.

4. The fusion protein of Claim 1, wherein the anti-TIGIT antibody is an animal antibody, chimeric antibody, or humanized antibody.

5. The fusion protein of Claim 1, wherein the antigen-binding fragment is scFv, scFv-Fc, (scFv)₂, Fab, Fab', or F(ab')₂ of the anti-TIGIT antibody.

6. The fusion protein of Claim 1, wherein the interleukin-15 comprises the amino acid sequence of SEQ ID NO: 38.

7. The fusion protein of Claim 1, wherein there is no peptide linker between the anti-TIGIT antibody or the antigen-binding fragment thereof and interleukin-15.

8. The fusion protein of Claim 1, wherein a peptide linker is comprised between the anti-TIGIT antibody or the antigen-binding fragment thereof and interleukin-15.

9. The fusion protein of Claim 8, wherein the peptide linker is [EAAAK]n (wherein n is an integer from 1 to 5, representing the number of [EAAAK]) or [(G)mS]1 (wherein m is an integer from 1 to 5, representing the number of G, and 1 is an integer from 1 to 5, representing the number of [(G)mS]).

10. A nucleic acid encoding the fusion protein of any one of Claims 1 to 9.

11. A recombinant vector comprising the nucleic acid of Claim 10.

12. A recombinant cell anchoring the nucleic acid of Claim 10 or a recombinant vector comprising the nucleic acid.

13. A pharmaceutical composition for preventing or treating a cancer, the composition comprising the fusion protein of any one of Claims 1 to 9.

14. The pharmaceutical composition of Claim 13, wherein the cancer is a solid tumor or hematologic cancer.

15. A pharmaceutical composition for immune enhancement, the composition comprising the fusion protein of any one of Claims 1 to 9.

16. A pharmaceutical composition for preventing or treating an immune-related disease, the composition comprising the fusion protein of any one of Claims 1 to 9.

17. The pharmaceutical composition of Claim 16, wherein the immune-related disease is an infectious disease, an inflammatory disease, or an autoimmune disease.
